# EUROPÄISCHE PATENTSCHRIFT

(11) **EP 3 129 352 B1**
(45) Veröffentlichungstag und Bekanntmachung des Hinweises auf die Patenterteilung: **15.11.2017**
(21) Anmeldenummer: 15717146.3
(22) Anmeldetag: 13.04.2015
(51) Int. Cl.: C07C 263/10, C07C 265/14, C08G 18/76, C08G 18/38, G02B 1/04, C08G 18/75, C08G 18/24, C08K 5/315

(54) **ZUSAMMENSETZUNG ENTHALTEND AROMATISCHE NITRILE ZUR HERSTELLUNG TRANSPARENTER POLYTHIOURETHANKÖRPER**
METHOD FOR MANUFACTURING XYLYLENE DIISOCYANATES IN THE GASEOUS PHASE
PROCÉDÉ DE FABRICATION DE DIISOCYANATES DE XYLYLÈNE EN PHASE GAZEUSE

(30) Priorität: 11.04.2014 EP 14164345
(43) Veröffentlichungstag der Anmeldung: 15.02.2017
(73) Patentinhaber: Covestro Deutschland AG, 51373 Leverkusen (DE)
(72) Erfinder: MALEIKA, Robert, 40589 Düsseldorf (DE); LANGENSTÜCK, Fredie, 51381 Leverkusen (DE); EGGERT, Christoph, 50733 Köln (DE); LATORRE MARTINEZ, Irene Cristina, 51373 Leverkusen (DE); STERN, Frank-Stefan, 51427 Bergisch Gladbach (DE); SANDERS, Josef, 51375 Leverkusen (DE)
(74) Vertreter: Levpat
(86) Internationale Anmeldenummer: PCT/EP2015/057960
(87) Internationale Veröffentlichungsnummer: WO 2015/155367

(56) Entgegenhaltungen:
- EP-A1- 1 928 928
- US-A1- 2006 155 093

## Beschreibung

Die vorliegende Erfindung betrifft Zusammensetzungen zur Herstellung transparenter Polythiourethankörper enthaltend oder bestehend aus
A) einer Polyisocyanatkomponente enthaltend zumindest ein Polyisocyanat mit einer Funktionalität an Isocyanatgruppen von wenigstens 2 pro Molekül,
B) einer Thiolkomponente enthaltend zumindest ein Polythiol mit einer Funktionalität an Thiolgruppen von wenigstens 2 pro Molekül,
   sowie gegebenenfalls
C) Hilfs- und Zusatzmittel,
wobei das Verhältnis von Isocyanatgruppen zu gegenüber Isocyanaten reaktiven Gruppen 0,5 : 1 bis 2,0 : 1 beträgt. Die Erfindung betrifft ferner ein Verfahren zur Herstellung transparenter Polythiourethankörper durch Umsetzung einer solchen Zusammensetzung und die so erzeugten Polythiourethankörper, sowie die Verwendung von aromatischen Nitrilen zur Herstellung transparenter Polythiourethankörper und zudem eine Mischung aus einem Polyisocyanat und einem aromatischen Nitril zur Herstellung kompakter transparenter Polythiourethankörper.

Für verschiedene Anwendungen, beispielsweise als leichtgewichtiger Ersatz für mineralisches Glas zur Herstellung von Scheiben für Automobil- und Flugzeugbau oder als Vergussmassen für optische, elektronische oder optoelektronische Bauteile, ist heute im Markt ein zunehmendes Interesse an transparenten, lichtechten Polyurethanmassen zu verzeichnen.

Insbesondere für hochwertige optische Anwendungen, wie z. B. für Linsen oder Brillengläser, besteht allgemein der Wunsch nach Kunststoffinaterialien, die eine hohe Lichtbrechung und gleichzeitig eine geringe Dispersion (hohe Abbe-Zahl) aufweisen.

Die Herstellung von transparenten Polyurethanmassen mit hohem Brechungsindex wurde bereits vielfach beschrieben. Oft kommen dabei als Polyisocyanatkomponenten sogenannte araliphatische Diisocyanate zum Einsatz, d. h. solche Diisocyanate, deren Isocyanatgruppen über aliphatische Reste an einem aromatischen System gebunden vorliegen. Aufgrund ihrer aromatischen Strukturen ergeben araliphatische Diisocyanate Polyurethane, die einen erhöhten Brechungsindex aufweisen, gleichzeitig garantieren die aliphatisch gebundenen Isocyanatgruppen die für hochwertige Anwendungen benötigte Lichtechtheit und geringe Vergilbungsneigung.

US-A 4680369 und US-A 4689387 beschreiben beispielsweise als Linsenmaterialien geeignete Polyurethane bzw. Polythiourethane, bei deren Herstellung zur Erzielung besonders hoher Brechungsindices spezielle Schwefel enthaltende Polyole bzw. mercaptofunktionelle aliphatische Verbindungen mit monomeren araliphatischen Diisocyanaten, wie z. B. 1,3-Bis(isocyanatomethyl)benzol (m-Xylylendiisocyanat, m-XDI), 1,4-Bis(isocyanatomethyl)benzol (p-Xylylendiisocyanat, p-XDI), 1,3-Bis(2-isocyanatopropan-2-yl)benzol (m-Tetramethylxylylendiisocyanat, m-TMXDI) oder 1,3-Bis(isocyanatomethyl)-2,4,5,6-tetrachlorbenzol kombiniert werden.

Monomere araliphatische Diisocyanate, wie m- und p-XDI oder m-TMXDI, werden auch in einer Vielzahl weiterer Veröffentlichungen, wie z. B. der EP-A 0 235 743, EP-A 0 268 896, EP-A 0 271 839, EP-A 0 408 459, EP-A 0 506 315, EP-A 0 586 091 und EP-A 0 803 743, als die bevorzugten Polyisocyanatkomponente zur Herstellung hochbrechender Linsenmaterialien genannt. Sie dienen dabei als Vernetzerkomponenten für Polyole und/oder Polythiole und ergeben in Abhängigkeit vom Reaktionspartner transparente Kunststoffe mit hohen Brechungsindices im Bereich von 1,56 bis 1,67 und vergleichsweise hohen Abbe-Zahlen bis zu 45.

Ein wesentlicher Nachteil der genannten Verfahren zur Herstellung hoch lichtbrechender Polyurethane beziehungsweise Polythiourethane für optische Anwendungen ist, dass die Reaktion zwischen Polythiolen und Polyisocyaten sehr empfindlich gegen Verunreinigungen ist, so dass die gefertigten Linsen zum Teil hinsichtlich ihrer Transparenz und Trübungsfreiheit nicht immer den gewünschten Anforderungen genügen. Dies gilt vor allem für die Anwendung in Linsen und dergleichen.

Die Aufgabe der vorliegenden Erfindung bestand somit darin, eine Zusammensetzung zur Herstellung transparenter Polythiourethankörper anzugeben, mit der sich solche Körper mit verbesserten optischen Eigenschaften, insbesondere einer verbesserten Transparenz und Trübungsfreiheit erzeugen lassen.

Diese Aufgabe wurde bei einer Zusammensetzung der eingangs genannten Art dadurch gelöst, dass die Zusammensetzung ferner D) zumindest ein aromatisches Nitril enthält.

Gegenstand der vorliegenden Erfindung ist damit eine Zusammensetzung zur Herstellung transparenter Polythiourethankörper enthaltend oder bestehend aus
A) einer Polyisocyanatkomponente enthaltend zumindest ein Polyisocyanat mit einer Funktionalität an Isocyanatgruppen von wenigstens 2 pro Molekül,
B) einer Thiolkomponente enthaltend oder bestehend aus zumindest ein Polythiol mit einer Funktionalität an Thiolgruppen von wenigstens 2 pro Molekül,
   sowie gegebenenfalls
C) Hilfs- und Zusatzmittel,
   wobei das Verhältnis von Isocyanatgruppen zu gegenüber Isocyanaten reaktiven Gruppen 0,5 : 1 bis 2,0 : 1 beträgt,
   wobei die Zusammensetzung dadurch gekennzeichnet ist, dass
   die Zusammensetzung ferner
D) zumindest ein aromatisches Nitril enthält.

Im Rahmen der vorliegenden Erfindung wird unter dem Begriff transparent verstanden, dass der transparente Körper bei einer Dicke von 2 mm und bei der Normlichtart D65 (definiert in DIN 6173) eine Transmission von ≥ 85 % aufweist. Wobei dieser Transmissionswert bei der optionalen Mitverwendung von UV-Stabilisatoren und Farbstoffen von dem vorstehend genannten Wert von ≥ 85 % abweichen kann.

Der vorliegenden Erfindung liegt die Erkenntnis zugrunde, dass durch die Anwesenheit wenigstens eines aromatischen Nitrils in Zusammensetzungen der eingangs genannten Art bei der Weiterverarbeitung zu Polythiourethanen deutlich seltener Trübungen in den so erzeugten Formkörpern auftreten. Ohne an eine Theorie gebunden zu sein wird vermutet, dass die Nitrile entweder mit den Thiolen reagieren können oder als Löslichkeitsvermittler für entstehende Dimere oder Trimere des Polyisocyanats wirken. Diese könnten ohne das Nitril ausfallen, was ein möglicher Grund für die auftretende Trübung sein kann.

Ein weiterer Vorteil besteht darin, dass die erfindungsgemäße Zusammensetzung weniger empfindlich gegen eingeschlepptes Wasser ist, als bislang bekannte Systeme, wie sie beispielsweise in US 8,044,165 B2 beschrieben sind. Normalerweise muss insbesondere die Thiolkomponente getrocknet werden, um überschüssiges Wasser zu entfernen, das ansonsten die optischen Eigenschaften erzeugter optischer Bauteile beeinträchtigen könnte. Demgegenüber ist dies bei der vorliegenden Erfindung nicht notwendig. So kann der Wassergehalt der Thiolkomponente bei den erfindungsgemäßen Zusammensetzungen > 600 ppm betragen, insbesondere > 800 ppm oder gar mehr als 900 ppm.

Es existieren verschiedene Möglichkeiten, das aromatische Nitril in die Zusammensetzung einzubringen. Die Nitrilzugabe kann bevorzugt bei der Formulierung der Zusammensetzung erfolgen, wobei das Nitril in besonders bevorzugter Weise mit der Polyisocyanatkomponente vorgemischt wird, bevor die Umsetzung mit der Thiolkomponente erfolgt. Die Erfindung ist jedoch keinesfalls auf diese Möglichkeit beschränkt. So kann die Nitrilzugabe auch vor oder während der Erzeugung der Polyisocyanate erfolgen, was typischerweise durch eine Flüssigphasen- oder Gasphasen-Phosgenierung der entsprechenden Polyamine erfolgt. Auch die Zugabe von Teilmengen des aromatischen Nitrils zu beliebigen Zeitpunkten ist möglich.

Es ist ferner möglich, Teilmengen oder die Gesamtmenge der aromatischen Nitrile erst beim Vermischen der reaktiven Komponenten zuzugeben oder mit der Thiolkomponente vorzumischen.

Ebenso kann - abgesehen von der vorbeschrieben aktiven Zugabe der aromatischen Nitrile - eine Teilmenge oder auch die Gesamtmenge an aromatischen Nitrilen "in-situ" erzeugt werden. Dies geschieht über eine Gasphasenphosgenierung araliphatischer Polyamine zu den entsprechenden araliphatischen Polyisocyanaten. Dabei entstehen - anders als bei der Flüssigphasenphosgenierung - aromatische Nitrile als Nebenprodukte der Phosgenierung. Ganz bevorzugt wird diese Möglichkeit bei der Gasphasenphosgenierung von 1,3-Xylyendiamin (1,3-XDA) und/oder 1,4-Xylyendiamin (1,4-XDA) zu 1,3-Bis(isocyanatomethyl)benzol (1,3 XDI) und/oder 1,4-Bis(isocyanatomethyl)benzol (1,4 XDI) angewendet. Je nach gewünschter Nitrilmenge kann das so erzeugte araliphatische Diisocyanat oder eine Mischung der genannten Verbindungen mit der produktionsbedingt enthaltenen Menge an aromatischen Nitrilen in einer erfindungsgemäßen Zusammensetzung eingesetzt, oder aber die Nitrilmenge durch Zugabe weiterer aromatischer Nitrile, die identisch oder verschieden zu den enthaltenen Nitrilen sein können, erhöht werden.

Grundsätzlich können die erfindungsgemäßen Zusammensetzungen neben den aromatischen Nitrilen zusätzlich auch andere organische Nitrile enthalten, wie beispielsweise aliphatische, cycloaliphatische oder araliphatische Nitrile.

Erfindungsgemäß ist vorgesehen, dass die Polyisocyanatkomponente zumindest ein Polyisocyanat enthält, das wenigstens zwei Isocyanatgruppen pro Molekül besitzt, insbesondere von 2 bis 6, vorzugsweise von 2 bis 4, besonders bevorzugt von 2 bis 3. Dabei können auch Mischungen von Polyisocyanaten unterschiedlicher Funktionalität eingesetzt werden, sodass sich ungeradzahlige mittlere Funktionalitäten ergeben. Unter einem Polyisocyanat werden im Rahmen der vorliegenden Erfindung organische Isocyanate verstanden mit zwei oder mehr freien Isocyanatgruppen. Das Polyisocyanat kann dabei in monomerer Form oder auch in oligomerer Form vorliegen. Hierfür geeignete Modifizierungsreaktionen sind beispielsweise die üblichen Verfahren zur katalytischen Oligomerisierung von Isocyanaten unter Bildung von Uretdion-, Isocyanurat-, Iminooxadiazindion- und/oder Oxadiazintrionstrukturen oder zur Biuretisierung von Diisocyanaten, wie sie z. B. in Laas et al., J. Prakt. Chem. 336, 1994, 185-200, in DE-A 1 670 666 und EP-A 0 798 299 beispielhaft beschrieben sind. Konkrete Beschreibungen solcher Polyisocyanate auf Basis araliphatischer Diisocyanate finden sich z. B. auch in EP-A 0 081 713, EP-A 0 197 543, GB-A 1 034 152 und JP-A 05286978. Damit bezieht sich die Silbe "poly" im Wesentlichen auf die Zahl der Isocyanatgruppen pro Molekül und bedeutet nicht zwangsläufig, dass das Polyisocyanat eine oligomere oder gar polymere Struktur aufweisen muss.

Im Prinzip können als Polyisocyanate im Rahmen der vorliegenden Erfindung sämtliche an sich bekannte Polyisocyanate eingesetzt werden. Dies sind beispielsweise solche des Molekulargewichtsbereiches 140 bis 400 g/mol, wie z. B. 1,4-Diisocyanatobutan, 1,5-Diisocyanatopentan, 1,6-Diisocyanatohexan (HDI), 1,5-Diisocyanato-2,2-dimethylpentan, 2,2,4- bzw. 2,4,4-Trimethyl-1,6-diisocyanatohexan, 1,8-Diisocyanatooctan, 1,9-Diisocyanato-nonan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methyl-cyclohexan, 1-Isocyanato-3,3,5-trimethyl-5-isocyanatomethylcyclohexan (Isophorondiisocyanat; IPDI), 1-Isocyanato-1-methyl-4(3)-isocyanatomethylcyclohexan, 2,4'- und 4,4'-Diisocyanatodicyclohexylmethan (H₁₂-MDI), 1,3- und 1,4-Bis(isocyanatomethyl)cyclohexan, 2,5-Bis(isocyanatomethyl)-bicyclo[2.2.1]heptan, 2,6-Bis(isocyanatomethyl)-bicyclo [2.2.1]heptan, 4,4'-Diisocyanato-3,3'-dimethyldicyclohexylmethan, 4,4' Diisocyanato-3,3',5,5'-tetramethyldicyclo-hexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanatoadamantan, 1,3-Dimethyl-5,7-diisocyanatoadamantan, 1,3- und 1,4-Bis-(isocyanatomethyl)benzol, 1,3-und 1,4-Bis(1-isocyanato-1-methylethyl)-benzol (TMXDI), Bis(4-(1-isocyanato-1-methylethyl)phenyl)-carbonat, 1,3- und 1,4-Phenylendiisocyanat, 2,4- und 2,6-Toluylendiisocyanat sowie beliebige Gemische dieser Isomeren, Diphenylmethan-2,4'-und/oder -4,4'-diisocyanat und Naphthylen-1,5-diisocyanat sowie beliebige Gemische solcher Diisocyanate.

Die Polyisocyanate können dabei durch beliebige Verfahren hergestellt sein, wie beispielsweise durch Phosgenierung der entsprechenden Amine in der Gas- oder Flüssigphase, aber auch durch Phosgen-freie Methoden wie beispielsweise durch Carbamatspaltung.

Vorzugsweise sind die Polyisocyanate ausgewählt aus 1,3-Bis(isocyanatomethyl)benzol (1,3 XDI), 1,4-Bis(isocyanatomethyl)benzol (1,4 XDI), 2,5-Bis(isocyanatomethyl)bicyclo[2.2.1]heptan, 2,6-Bis(isocyanatomethyl)bicyclo[2.2.1]heptan, 1,4-Bis(isocyanatomethyl)cyclohexan, 1,3-Bis(isocyanatomethyl)cyclohexan, 2,2'-Diisocyanatodicyclohexylmethan, 2,4'-Diisocyanatodicyclohexylmethan, 4,4'-Diisocyanatodicyclohexylmethan (H12 MDI), 1-Isocyanato-3,3,5-trimethyl-5-isocyanatomethylcyclohexan (Isophorondiisocyanat; IPDI) oder Mischungen von diesen, insbesondere bevorzugt 1,3-Bis(isocyanatomethyl)benzol (1,3 XDI) und/ oder 1,4-Bis(isocyanatomethyl)benzol (1,4 XDI). Diese Poly- beziehungsweise Diisocyanate sind bevorzugt, weil hierdurch Linsenmaterialien mit besonders guten optischen Eigenschaften erhältlich sind, wie hohem Brechungsindex und hoher Transparenz. Diese Vorteile sind bei den als bevorzugt genannten Diisocyanaten 1,3-Bis(isocyanatomethyl)benzol (1,3 XDI) und 1,4-Bis(isocyanatomethyl)benzol (1,4 XDI) besonders ausgeprägt.

Die mittlere Isocyanatfunktionalität der Polyisocyanatkomponente A) beträgt bevorzugt 1,8 bis 4,0, insbesondere 1,9 bis 3,5, besonders bevorzugt 2,0 bis 3,0.

Nach der vorliegenden Erfindung kann vorgesehen sein, dass wenigstens ein Polyisocyanat der Polyisocyanatkomponente A) durch Gasphasenphosgenierung aliphatischer, cycloaliphatischer, aromatischer oder araliphatischer Polyamine hergestellt ist. Ein derartiges Verfahren für die Erzeugung der erfindungsgemäß einzusetzenden Polyisocyanate ist in EP 1 754 698 B1 beschrieben. Ebenfalls einsetzbar ist ein Verfahren zur Gasphasenphosgenierung, wie es in WO 2013/079517 A1 offenbart ist.

Bei der Gasphasenphosgenierung wird vorzugsweise so vorgegangen, dass die entsprechenden Ausgangssubstanzen, das heißt die den herzustellenden Polyisocyanaten zugrundeliegenden Polyamine, gegebenenfalls unter Zusatz von Stabilisatoren und / oder eines Inertgases, verdampft werden, dabei wird wenn benötigt bei einem Druck von < 1000 mbar gearbeitet. Die auf diese Weise verdampften Polyamine werden in einem Kreislauf mit einer mittleren Verweilzeit von 5 bis 90 Minuten und einer Temperatur von 40 bis 190 °C geführt und dabei bei einer Temperatur von 10 bis 100 K oberhalb der Verdampfungstemperatur der Amine unter dem gegebenen Druck nach an sich bekannten Verfahren der Gasphasenphosgenierung mit Phosgen umgesetzt. Die vorgenannten Verfahrensbedingungen gelten insbesondere für die Erzeugung von 1,3-Xylylendiisocyanat und 1,4-Xylylendiisocyanat. Auf diese Weise entstehen vergleichsverweise hohe Mengen an Nitrilen, die sich vorteilhaft bei der Weiterverarbeitung in einer erfindungsgemäßen Zusammensetzung auswirken, insbesondere im Hinblick auf die Transparenz daraus erzeugter Formkörper. In diesem Falle kann - je nach gewünschtem Gehalt an aromatischen Nitrilen in der Zusammensetzung kann bei dieser Ausführungsform auf eine aktive Zugabe aromatischer Nitrile verzichtet werden.

Geeignete Modifizierungsreaktionen zur Herstellung der Polyisocyanatkomponenten A) sind, sofern gewünscht, eine Urethanisierung und/oder Allophanatisierung araliphatischer Diisocyanate nach Zusatz molar unterschüssiger Mengen hydroxyfunktioneller Reaktionspartner, insbesondere niedermolekularer ein- oder mehrwertiger Alkohole des Molekulargewichtsbereiches 32 bis 300 g/mol, wie z. B. Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole, Hydroxymethylcyclohexan, 3-Methyl-3-hydroxymethyloxetan, 1,2-Ethandiol, 1,2- und 1,3-Propandiol, die isomeren Butandiole, Pentandiole, Hexandiole, Heptandiole und Octandiole, 1,2- und 1,4-Cyclohexandiol, 1,4-Cyclohexandimethanol, 4,4'-(1-Methylethyliden)-biscyclohexanol, Diethylenglylol, Dipropylenglykol, 1,2,3-Propantriol, 1,1,1-Trimethylolethan, 1,2,6-Hexantriol, 1,1,1-Trimethylolpropan, 2,2-Bis(hydroxymethyl)-1,3-propandiol oder 1,3,5-Tris(2-hydroxyethyl)-isocyanurat, oder beliebiger Gemische solcher Alkohole. Bevorzugte Alkohole zur Herstellung Urethan- und/oder Allophanat-modifizierter Polyisocyanatkomponenten A) sind die genannten Monoalkohole und Diole mit 2 bis 8 Kohlenstoffatomen.

Konkrete Beschreibungen Urethan- und/oder Allophanat-modifizierter Polyisocyanate auf Basis araliphatischer Diisocyanate finden sich beispielsweise in EP-A 1 437 371, EP-A 1 443 067, JP-A 200516161691, JP-A 2005162271.

Soll für die erfindungsgemäße Zusammensetzung ein oder mehrere, über Gasphasenphosgenierung erzeugte Polyisocyanate eingesetzt werden, enthält nach einer bevorzugten Ausgestaltung der erfindungsgemäßen Zusammensetzung die Polyisocyanatkomponente wenigstens 30 Gew.-% bezogen auf die Polyisocyanatkomponente an durch Gasphasenphosgenierung hergestellten Polyisocyanat, insbesondere wenigstens 50 Gew.-%, weiter bevorzugt wenigstens 80 Gew.-% oder gar wenigstens 90 Gew.-%, besonders bevorzugt wenigstens 95 Gew.-% oder gar wenigstens 96 oder wenigstens 98 Gew.-%.

Bei dem Einsatz von über Gasphasenphosgenierung erzeugtem Polyisocyanat bildet bevorzugt wenigstens ein Nitril einen weiteren Bestandteil der Polyisocyanatkomponente, das als Nebenprodukt in der über Gasphasenphosgenierung enthaltenen Polyisocyanatkomponente enthalten ist. Neben dem über Gasphasenphosgenierung erzeugten Polyisocyanat kann der Polyisocyanatkomponente durchaus auch ein über andere Herstellungswege erzeugtes Polyisocyanat zugesetzt sein, wie beispielsweise ein Polyisocyanat, welches über Flüssigphasen-Phosgenierung erzeugt wurde.

Je nach Nitril-Gehalt der Polyisocyanatkomponente kann der Anteil an durch andere als Gasphasenphosgenierung erzeugten Polyisocyanaten beispielsweise bis zu 19 Gew.-% bezogen auf die Polyisocyanatkomponente A) betragen, gegebenenfalls auch bis zu 14 Gew.-%, bis zu 9 Gew.-%., bis zu 4 Gew.-% oder bis zu 3 Gew.-%.

Nach einer bevorzugten Ausführungsform der erfindungsgemäßen Zusammensetzung, in der zumindest ein über Gasphasenphosgenierung erzeugtes Polyisocyanat eingesetzt wird, enthält die Polyisocyanatkomponente wenigstens 0,005 Gew.-% bezogen auf die Polyisocyanatkomponente A) an wenigstens einem aromatischen Nitril, insbesondere wenigstens 0,01 Gew.-%, bevorzugt 0,005 bis 15 Gew.-%, weiter bevorzugt 0,01 bis 5 Gew.-%, besonders bevorzugt 0,1 bis 2 Gew.-% oder gar 0,1 bis 1 Gew.-%.

Wie bereits vorstehend ausgeführt wurde, können die erfindungsgemäß eingesetzten und durch Gasphasenphosgenierung erzeugten Polyisocyanatkomponenten herstellungsbedingt bereits einen gewissen Anteil an Nitrilen enthalten, der unter anderem durch die Natur der bei der Reaktion eingesetzten Polyamine und darüber hinaus durch die Wahl der Reaktionsbedingungen, insbesondere den Druck und die Reaktionstemperatur, beeinflusst werden kann. Dies gilt insbesondere für die im Rahmen der vorliegenden Erfindung besonders bevorzugten Polyisocyanate 1,3-Bis(isocyanatomethyl)benzol (1,3 XDI) und 1,4-Bis(isocyanatomethyl)benzol (1,4 XDI), die nach der Gasphasenphosgenierung in der Regel vergleichsweise hohe Gehalte an entsprechenden Nitrilen aufweisen.

Unabhängig von der Anwesenheit eines oder mehrerer über Gasphasenphosgenierung erzeugter Polyisocyanate enthält die Zusammensetzung in bevorzugter Weise 0,0025 bis 10 Gew.-%, bezogen auf die gesamte Zusammensetzung an aromatischem Nitril, insbesondere 0,005 bis 5 Gew.-%, weiter bevorzugt 0,01 bis 3 Gew.-% und besonders bevorzugt 0,05 bis 2 Gew.-%. Diese Einsatzmengen an aromatischen Nitrilen - unabhängig ob sie als Nebenprodukt der Gasphasenphosgenierung vorliegen oder vollständig oder teilweise aktiv zugesetzt wurden - sind besonders vorteilhaft, weil sich hierdurch besonders transparente und trübungsfreie optische Formkörper aus Polythiourethan herstellen lassen, wie beispielsweise Linsen.

Der Gehalt an Nitrilen wird im Rahmen der vorliegenden Erfindung über Derivatisierung mit Diethylamin und anschließender HPLC-MS durch Integration der Flächen der Signale im UV bestimmt. Für die HPLC-MS Messung kann beispielsweise das folgende Programm gewählt werden:
Synapt G2-S HR-MS, ACQUITY UPLC (Waters) QS. Nr.: 02634
UV: PDA (Total Absorbance Chromatogram)
Säule: Kinetex 100 x 2.1mm_1.7µm
Säulentemperatur: 30 °C

Die mobile Phase bestand aus:
Lösemittel:
   A) Wasser + 0.05% Ameisensäure
   B) Acetonitril + 0.05% Ameisensäure
Flussrate: 0.5 ml/min
Gradient: t0/5%B_t0.5/5%B_t6/100%B_t7/100%B_t7.1/5%B_t8/5%B

Im Rahmen der vorliegenden Erfindung ist es bevorzugt, dass das Nitril von demselben Polyamin abgeleitete ist wie das Polyisocyanat. Dies gilt dabei sowohl für die herstellungsbedingt enthaltenen Nitrile als auch für gegebenenfalls nachträglich zugefügte Nitrile. Dies ist besonders vorteilhaft, da aufgrund des gleichen chemischen Grundgerüst wie das des bei der Gasphasenphosgenierung erzeugten Polyisocyanats eine besonders gute Vermittlungswirkung durch das Nitril erreicht wird.

Besonders bevorzugt ist es ferner, dass das Nitril wenigstens eine weitere funktionelle Gruppe enthält, wobei es sich insbesondere um eine Gruppe handelt, die in das Polythiourethan Netzwerk einbauen kann, um eine spätere Migration des Nitrils aus dem Formkörper zu verhindern, und besonders bevorzugt um eine Isocyantgruppe handeln kann. Dies soll im Folgenden anhand des Beispiels 1,3-Bis(Isocyanatomethyl)benzol(1,3-XDI) verdeutlicht werden. Dieses Diisocyanat kann durch Gasphasenphosgenierung von 1,3-Bis(Aminomethyl)benzol erzeugt werden. Das besonders bevorzugte Isocyanatonitril ist in diesem Fall das 3-(Isocyantomethyl)benzonitril.

Bei der Umsetzung eines solchen Isocyanatonitrils mit der Thiolkomponente können Nitril-modifizierte Thiourethane entstehen. Dies sei gezeigt am Beispiel des 3-(Isocyantomethyl)benzonitrils:

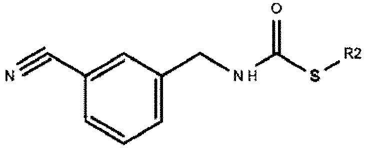

Darin bezeichnet R2 den Rest des eingesetzten Polythiols. Für 4-Mercaptomethyl-1,8-dimercapto-3,6-dithiaoctan (DMPT) entspricht der Rest R2 folgender Struktureinheit, wobei der linke Bindungsstrich in der Formel die Bindung zum Schwefelatom in der vorgenannten Struktur darstellt:

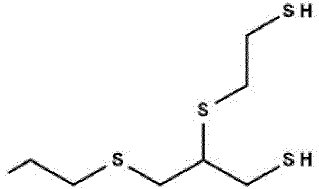

Es können auch beide Thiolgruppen des DMPT mit jeweils einem Molekül 3-(Isocyantomethyl)benzonitril reagieren, wobei die folgende Struktur entsteht:

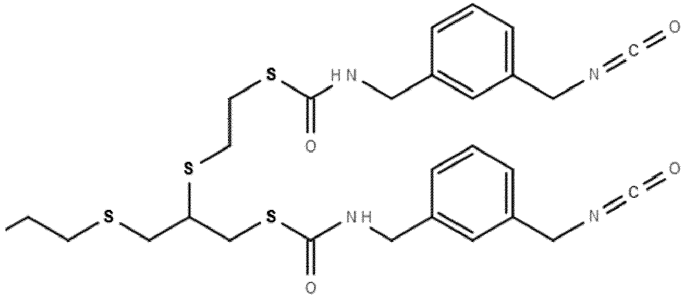

Analoge Verbindungen können auch mit optional eingesetzten Polyolen entstehen. Zunächst allgemein das Reaktionsprodukt von 3-(Isocyantomethyl)benzonitril mit einem Polyol mit R1 als Rest des eingesetzten Polyols:

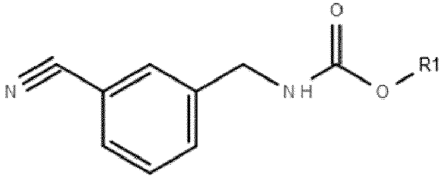

Es entsteht also ein Nitril-modifiziertes Urethan. Bei Einsatz eines Diols können auch beide OH-Gruppen mit jeweils einem Molekül 3-(Isocyantomethyl)benzonitril reagieren, wobei die folgende Struktur entsteht:

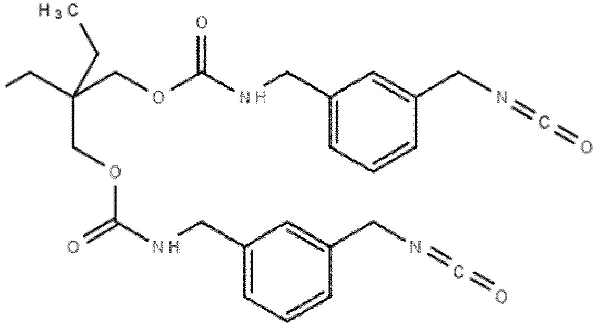

Unter aromatischen Nitrilen werden im Sinne dieser Erfindung Verbindungen verstanden, die direkt an einen Aromaten gebunden eine Nitrilgruppe aufweisen und bevorzugt eine Molmasse von < 1000 g/mol, besonders bevorzugt < 500 g/mol haben.

Nach einer bevorzugten Ausgestaltung der erfindungsgemäßen Zusammensetzung ist das Nitril ausgewählt aus Benzonitril, 3-(Isocyanatomethyl)benzonitril, 4-(Isocyanatomethyl)benzonitril, 3-(Chloromethyl)benzonitril, 4-(Chloromethyl)benzonitril, 3-Cyanobenzoesäure, 4-Cyanobenzoesäure, 2-Hydroxybenzonitril, 3- Hydroxybenzonitril, 4-Hydroxybenzonitril, 3-Cyanobenzoesäurechlorid, 4-Cyanobenzoesäurechlorid oder Mischungen von diesen, insbesondere Benzonitril, 3-(Chloromethyl)benzonitril, 4-(Chloromethyl)benzonitril, 3-(Isocyanatomethyl)benzonitril, und/ oder 4-(Isocyanatomethyl)benzonitril, besonders bevorzugt 3-(Isocyanatomethyl)benzonitril, und/ oder 4-(Isocyanatomethyl)benzonitril. Der Einsatz dieser Nitrile ist insbesondere bei Einsatz einer Polyisocyanatkomponente von Vorteil, die größere Anteile an 1,3-XDI und/ oder 1,4-XDI, wie > 50 Gew.-% bezogen auf die Polyisocyanatkomponente A) aufweist, oder die hinsichtlich der Polyisocyanate vollständig aus diesen Verbindungen besteht. In diesen Fällen kann eine besonders ausgeprägte Verbesserung der optischen Eigenschaften eines daraus erzeugten Polythiourethankörpers beobachtet werden, speziell was dessen Transparenz angeht.

Nach einer besonders bevorzugten Ausführungsform der erfindungsgemäßen Zusammensetzung ist das Polyisocyanat ausgewählt aus 1,3-Bis(Isocyanatomethyl)benzol (1,3 XDI) und das Nitril aus 3-(Isocyanatomethyl)benzonitril und/oder dass das Polyisocyanat ausgewählt ist aus 1,4-Bis(isocyanatomethyl)benzol (1,4 XDI) und das Nitril aus 4-(Isocyanatomethyl)benzonitril.

Die erfindungsgemäße Zusammensetzung enthält ferner eine Thiolkomponente B). Diese beinhaltet oder besteht aus zumindest einem Polythiol mit einer Funktionalität an Thiolgruppen von wenigstens zwei pro Molekül, insbesondere von 2 bis 6, vorzugsweise von 2 bis 4, besonders bevorzugt von 3 bis 4. Dabei können auch Mischungen von Polythiolen unterschiedlicher Funktionalität eingesetzt werden, sodass sich ungeradzahlige mittlere Funktionalitäten ergeben. Unter einem Polythiol werden im Rahmen der vorliegenden Erfindung organische Thiole mit zwei oder mehr Thiolgruppen verstanden. Damit bezieht sich die Silbe "poly" im Wesentlichen auf die Zahl der Thiolgruppen und bedeutet, wie auch vorstehend bei den Polyisocyanat-Verbindungen ausgeführt nicht zwangsläufig, dass das Polythiol eine oligomere oder gar polymere Struktur aufweisen muss. Obgleich können die erfindungsgemäß eingesetzten Polythiole durchaus auch ein Polyehter-Grundgerüst, ein Polythiolether-Grundgerüst oder ein gemischtes Grundgerüst aus O-Ether und S-Ether-Einheiten besitzen.

Das Polythiol kann ein mittleres Molekulargewicht von 80 (Methandithiol) bis etwa 12000 g/mol aufweisen, vorzugsweise 250 bis 8000 g/mol.

Als Polythiole eignen sich beispielsweise Methandithiol, 1,2-Ethandithiol, 1,1-Propandithiol, 1,2-Propandithiol, 1,3-Propandithiol, 2,2-Propandithiol, 1,4-Butandithiol, 2,3-Butandithiol, 1,5-Pentandithiol, 1,6-Hexandithiol, 1,2,3-Propantrithiol, 1,1-Cyclohexandithiol, 1,2-Cyclohexandithiol, 2,2-Dimethylpropan-1,3-dithiol, 3,4-Dimethoxybutan-1,2-dithiol und 2-Methylcyclohexan-2,3-dithiol, Thioethergruppen enthaltende Polythiole, wie z. B. 2,4-Dimercaptomethyl-1,5-dimercapto-3-thiapentan, 4-Mercaptomethyl-1,8-dimercapto-3,6-dithiaoctan, 4,8-Dimercaptomethyl-1,11-dimercapto-3,6,9-trithiaundecan, 4,7-Dimercaptomethyl-1,11-dimercapto-3,6,9-trithiaundecan, 5,7-Dimercaptomethyl-1,11-dimercapto-3,6,9-trithiaundecan, 4,5-Bis(mercaptoethylthio)-1,10-dimercapto-3,8-dithiadecan, Tetrakis(mercaptomethyl)methan, 1,1,3,3-Tetrakis(mercaptomethylthio)propan, 1,1,5,5-Tetrakis(mercaptomethylthio)-3-thiapentan,1,1,6,6-Tetrakis(mercaptomethylthio)-3,4-dithiahexan, 2-Mercaptoethylthio-1,3-dimercaptopropan, 2,3-Bis(mercaptoethylthio)-1-mercaptopropan, 2,2-Bis(mercaptomethyl)-1,3-dimercaptopropan, Bis(mercaptomethyl)sulfid, Bis(mercaptomethyl)disulfid, Bis(mercaptoethyl)sulfid, Bis(mercaptoethyl)disulfid, Bis(mercaptopropyl)sulfid, Bis(mercaptopropyl)disulfid, Bis(mercaptomethylthio)methan, Tris(mercaptomethylthio)methan, Bis(mercaptoethylthio)methan, Tris(mercaptoethylthio)methan, Bis(mercaptopropylthio)methan, 1,2-Bis(mercaptomethylthio)ethan, 1,2-Bis(mercaptoethylthio)ethan, 2-Mercaptoethylthio)ethan, 1,3-Bis(mercaptomethylthio)propan, 1,3-Bis(mercaptopropylthio)propan, 1,2,3-Tris(mercaptomethylthio)propan, 1,2,3-Tris(mercaptoethylthio)propan, 1,2,3-Tris(mercaptopropylthio)propan, Tetrakis(mercaptomethylthio)methan, Tetrakis(mercaptoethylthiomethyl)methan, Tetrakis(mercaptopropylthiomethyl)methan, 2,5-Dimercapto-1,4-dithian, 2,5-Bis(mercaptomethyl)-1,4-dithian und dessen gemäß JP-A 07118263 erhältliche Oligomere, 1,5-Bis(mercaptopropyl)-1,4-dithian, 1,5-Bis(2-mercaptoethylthiomethyl)-1,4-dithian, 2-Mercaptomethyl-6-mercapto-1,4-dithiacycloheptan, 2,4,6-Trimercapto-1,3,5-trithian, 2,4,6-Trimercaptomethyl-1,3,5-trithian und 2-(3-Bis(mercaptomethyl)-2-thiapropyl)-1,3-dithiolan, Polyesterthiole, wie z. B. Ethylenglycol-bis(2-mercaptoacetat), Ethylenglycol-bis(3-mercaptopropionat), Diethylenglycol(2-mercaptoacetat), Diethylenglycol(3-mercaptopropionat), 2,3-Dimercapto-1-propanol(3-mercaptopropionat), 3-Mercapto-1,2-propandiol-bis(2-mercaptoacetat), 3-Mercapto-1,2-propandiol-bis(3-mercaptopropionat), Trimethylolpropan-tris(2-mercaptoacetat), Trimethylolpropan-tris(3-mercaptopropionat), Trimethylolethan-tris(2-mercaptoacetat), Trimethylolethan-tris(3-mercaptopropionat), Pentaerythrittetrakis(2-mercaptoacetat), Pentaerythrit-tetrakis(3-mercaptopropionat), Glycerin-tris(2-mercaptoacetat), Glycerin-tris(3-mercaptopropionat), 1,4-Cyclohexandiol-bis(2-mercaptoacetat), 1,4-Cyclohexandiol-bis(3-mercaptopropionat), Hydroxymethylsulfidbis(2-mercaptoacetat), Hydroxymethylsulfid-bis(3-mercaptopropionat), Hydroxyethylsulfid(2-mercaptoacetat), Hydroxyethylsulfid(3-mercaptopropionat), Hydroxymethyldisulfid(2-mercaptoacetat), Hydroxymethyldisulfid(3-mercaptopropionat), (2-Mercaptoethylester)thioglycolat und Bis(2-mercaptoethylester)thiodipropionat sowie aromatische Thioverbindungen, wie z. B. 1,2-Dimercaptobenzol, 1,3-Dimercaptobenzol, 1,4-Dimercaptobenzol, 1,2-Bis(mercaptomethyl)benzol, 1,4-Bis(mercaptomethyl)benzol, 1,2-Bis(mercaptoethyl)benzol, 1,4-Bis(mercaptoethyl)benzol, 1,2,3-Trimercaptobenzol, 1,2,4-Trimercaptobenzol, 1,3,5-Trimercaptobenzol, 1,2,3-Tris(mercaptomethyl)benzol, 1,2,4-Tris(mercaptomethyl)benzol, 1,3,5-Tris(mercaptomethyl)benzol, 1,2,3-Tris(mercaptoethyl)benzol, 1,3,5-Tris(mercaptoethyl)benzol, 1,2,4-Tris(mercaptoethyl)benzol, 2,5-Toluoldithiol, 3,4-Toluoldithiol, 1,4-Naphthalindithiol, 1,5-Naphthalindithiol, 2,6-Naphthalindithiol, 2,7-Naphthalindithiol, 1,2,3,4-Tetramercaptobenzol, 1,2,3,5-Tetramercaptobenzol, 1,2,4,5-Tetramercaptobenzol, 1,2,3,4-Tetrakis(mercaptomethyl)benzol, 1,2,3,5-Tetrakis(mercaptomethyl)benzol, 1,2,4,5-Tetrakis(mercaptomethyl)benzol, 1,2,3,4-Tetrakis(mercaptoethyl)benzol, 1,2,3,5-Tetrakis(mercaptoethyl)benzol, 1,2,4,5-Tetrakis(mercaptoethyl)benzol, 2,2'-Dimercaptobiphenyl und 4,4'-Dimercaptobiphenyl.

Vorzugsweise ist das Polythiol ausgewählt aus 4-Mercaptomethyl-1,8-dimercapto-3,6-dithiaoctan, 2,5-Bismercaptomethyl-1,4-dithian, 1,1,3,3-Tetrakis(mercaptomethylthio)propan, 5,7-Dimercaptomethyl-1,11-dimercapto-3,6,9-trithiaundecan, 4,7-Dimercaptomethyl-1,11-dimercapto-3,6,9-trithiaundecan, 4,8-Dimercaptomethyl-1,11-dimercapto-3,6,9-trithiaundecan, Trimethylolpropan-tris(3-mercaptopropionat), Trimethylolethan-tris(2-mercaptoacetat), Pentaerythrit-tetrakis(2-mercaptoacetat) und/ oder Pentaerythrit-tetrakis(3-mercaptopropionat).

Abgesehen von der Thiolkomponente B) kann die erfindungsgemäße Zusammensetzung auch andere, üblicherweise mit Polyisocyanaten reagierende Komponenten enthalten. Hierbei handelt es sich insbesondere um die üblichen aus der Polyurethanchemie bekannten Polyetherpolyole, Polyesterpolyole, Polyetherpolyesterpolyole, Polythioetherpolyole, polymermodifizierte Polyetherpolyole, Pfropfpolyetherpolyole, insbesondere solche auf Styrol- und/oder Acrylnitrilbasis, Polyetherpolyamine, hydroxylgruppenhaltigen Polyacetale und/oder hydroxylgruppenhaltigen aliphatischen Polycarbonate, die üblicherweise ein gewichtsmittleres Molekulargewicht von 106 bis 12000 g/mol, vorzugsweise 250 bis 8000 g/mol aufweisen. Ein breiter Überblick über geeignete Reaktionspartner B) findet sich beispielsweise in N. Adam et al.: "Polyurethanes", Ullmann's Encyclopedia of Industrial Chemistry, Electronic Release, 7th ed., chap. 3.2 - 3.4, Wiley-VCH, Weinheim 2005.

Unter einem Polythiourethan wird im Sinne der vorliegenden Erfindung ein Polymer verstanden, bei dem mehr als die Hälfte bis sämtliche der Bindungen zwischen dem Polyisocyanat und der oder den Isocyanat-reaktiven Komponente(n) Thiourethangruppen sind. Es können also zu weniger als die Hälfte andere Bindungen vorhanden sein, wie beispielsweise Urethan- oder Harnstoffbrücken. In diesem Falle enthält die erfindungsgemäße Zusammensetzung noch andere Isocyanat-reaktiven Komponente(n). Diese lediglich optionalen Komponenten werden im Folgenden näher beschrieben.

Geeignete Polyetherpolyole, sofern verwendet, sind beispielsweise solche der in der DE-A 2 622 951, Spalte 6, Zeile 65 - Spalte 7, Zeile 47, oder der EP-A 0 978 523 Seite 4, Zeile 45 bis Seite 5, Zeile 14 genannten Art, sofern sie den oben gemachten Angaben hinsichtlich Funktionalität und Molekulargewicht entsprechen. Besonders bevorzugte Polyetherpolyole B) sind Anlagerungsprodukte von Ethylenoxid und/oder Propylenoxid an Glycerin, Trimethylolpropan, Ethylendiamin und/oder Pentaerythrit.

Geeignete Polyesterpolyole, sofern verwendet, sind beispielsweise solche der in der EP-A 0 978 523 Seite 5, Zeilen 17 bis 47 oder der EP-A 0 659 792 Seite 6, Zeilen 8 bis 19 genannten Art, sofern sie den oben gemachten Angaben entsprechen, bevorzugt solche, deren Hydroxylzahl von 20 bis 650 mg KOH/g beträgt.

Geeignete Polyacetalpolyole, sofern verwendet, sind beispielsweise die bekannten Umsetzungsprodukte einfacher Glykole, wie z. B. Diethylenglykol, Triethylenglykol, 4,4'-Dioxethoxy-diphenyl-dimethylmethan (Addukt von 2 mol Ethylenoxid an Bisphenol A) oder Hexandiol, mit Formaldehyd oder auch durch Polykondensation cyclischer Acetale, wie z. B. Trioxan, hergestellte Polyacetale.

Ebenfalls können Aminopolyether oder Gemische von Aminopolyethern geeignet sein, d. h. Polyether mit gegenüber Isocyanatgruppen reaktionsfähigen Gruppen, die sich zumindest zu 50 Äquivalent-%, vorzugsweise zumindest zu 80 Äquivalent-%, aus primären und/oder sekundären, aromatisch oder aliphatisch gebundenen Aminogruppen und zum Rest aus primären und/oder sekundären, aliphatisch gebundenen Hydroxylgruppen zusammensetzen. Geeignete derartige Aminopolyether sind beispielsweise die in EP-A 0 081 701, Spalte 4, Zeile 26 bis Spalte 5, Zeile 40, genannten Verbindungen. Ebenfalls als Ausgangskomponente E) geeignet sind aminofunktionelle Polyetherurethane oder -harnstoffe, wie sie sich beispielsweise nach dem Verfahren der DE-A 2 948 419 durch Hydrolyse von isocyanatfunktionellen Polyetherprepolymeren herstellen lassen oder auch Aminogruppen aufweisende Polyester des obengenannten Molekulargewichtsbereichs.

Weitere geeignete gegenüber Isocyanatgruppen reaktive Komponenten sind beispielsweise auch die in EP-A 0 689 556 und EP-A 0 937 110 beschriebenen, z. B. durch Umsetzung epoxidierter Fettsäureester mit aliphatischen oder aromatischen Polyolen unter Epoxidringöffung erhältlichen speziellen Polyole.

Auch Hydroxylgruppen enthaltende Polybutadiene können gegebenenfalls eingesetzt werden.

Darüber hinaus sind als gegenüber Isocyanatgruppen reaktive Komponenten auch Schwefel enthaltende Hydroxyverbindungen geeignet. Beispielhaft seien hier genannt einfache Mercaptoalkohole, wie z. B. 2-Mercaptoethanol, 3-Mercaptopropanol, 1,3-Dimercapto-2-propanol, 2,3-Dimercaptopropanol und Dithioerythritol, Thioetherstrukturen enthaltende Alkohole, wie z. B. Di(2-hydroxyethyl)sulfid, 1,2-Bis(2-hydroxyethylmercapto)ethan, Bis(2-hydroxyethyl)disulfid und 1,4-Dithian-2,5-diol, oder Schwefel enthaltende Diole mit Polyesterurethan-, Polythioesterurethan-, Polyesterthiourethan- oder Polythioesterthiourethanstruktur der in der EP-A 1 640 394 genannten Art.

Als gegenüber Isocyanaten reaktive Verbindungen können die erfindungsgemäßen Zusammensetzungen auch niedermolekulare, hydroxy- und/oder aminofunktionelle Komponenten enthalten, d. h. solche eines Molekulargewichtsbereiches von 60 bis 500 g/mol, vorzugsweise von 62 bis 400 g/mol.

Hierbei handelt es sich beispielsweise um einfache ein- oder mehrwertige Alkohole mit 2 bis 14, vorzugsweise 4 bis 10 Kohlenstoffatomen, wie z. B. 1,2-Ethandiol, 1,2- und 1,3-Propandiol, die isomeren Butandiole, Pentandiole, Hexandiole, Heptandiole und Octandiole, 1,10-Decandiol, 1,2- und 1,4-Cyclohexandiol, 1,4-Cyclohexandimethanol, 4,4'-(1-Methylethyliden)-biscyclohexanol, 1,2,3-Propantriol, 1,1,1-Trimethylolethan, 1,2,6-Hexantriol, 1,1,1-Trimethylolpropan, 2,2-Bis(hydroxymethyl)-1,3-propandiol, Bis-(2-hydroxyethyl)-hydrochinon, 1,2,4- und 1,3,5-Trihydroxycyclohexan oder 1,3,5-Tris(2-hydroxyethyl)-isocyanurat.

Beispiele für geeignete niedermolekulare aminofunktionelle Verbindungen sind aliphatische und cycloaliphatische Amine und Aminoalkohole mit primär und/oder sekundär gebundenen Aminogruppen, wie z. B. Cyclohexylamin, 2-Methyl-1,5-pentandiamin, Diethanolamin, Monoethanolamin, Propylamin, Butylamin, Dibutylamin, Hexylamin, Monoisopropanolamin, Diisopropanolamin, Ethylendiamin, 1,3-Diaminopropan, 1,4-Diaminobutan, Isophorondiamin, Diethylentriamin, Ethanolamin, Aminoethylethanolamin, Diaminocyclohexan, Hexamethylendiamin, Methyliminobispropylamin, Iminobispropylamin, Bis(aminopropyl)piperazin, Aminoethylpiperazin, 1,2-Diaminocyclohexan, Triethylentetramin, Tetraethylenpentamin, 1,8-p-Diaminomenthan, Bis(4-aminocyclohexyl)methan, Bis(4-amino-3-methylcyclohexyl)methan, Bis(4-amino-3,5-dimethylcyclohexyl)methan, Bis(4-amino-2,3,5-trimethylcyclohexyl)methan, 1,1-Bis(4-aminocyclohexyl)propan, 2,2-Bis(4-aminocyclohexyl)propan, 1,1-Bis(4-aminocyclohexyl)ethan, 1,1-Bis(4-aminocyclohexyl)butan, 2,2-Bis(4-aminocyclohexyl)butan, 1,1-Bis(4-amino-3-methylcyclohexyl)ethan, 2,2-Bis(4-amino-3-methylcyclohexyl)propan, 1,1-Bis(4-amino-3,5-dimethylcyclohexyl)ethan, 2,2-Bis(4-amino-3,5-dimethylcyclohexyl)propan, 2,2-Bis(4-amino-3,5-dimethylcyclohexyl)butan, 2,4-Diaminodicyclohexylmethan, 4-Aminocyclohexyl-4-amino-3-methylcyclohexylmethan, 4-Amino-3,5-dimethylcyclohexyl-4-amino-3-methylcyclohexylmethan und 2-(4-Aminocyclohexyl)-2-(4-amino-3-methylcyclohexyl)methan.

Beispiele für aromatische Polyamine, insbesondere Diamine, mit Molekulargewichten unter 500, die geeignete gegenüber Isocyanaten reaktive Verbindungen B) darstellen, sind z. B. 1,2- und 1,4-Diaminobenzol, 2,4- und 2,6-Diaminotoluol, 2,4'- und/oder 4,4'-Diaminodiphenylmethan, 1,5-Diaminonaphthalin, 4,4',4"-Triaminotriphenylmethan, 4,4'-Bis-(methylamino)-diphenylmethan oder 1-Methyl-2-methylamino-4-aminobenzol. 1-Methyl-3,5-diethyl-2,4-diaminobenzol, 1-Methyl-3,5-diethyl-2,6-diaminobenzol, 1,3,5-Trimethyl-2,4-diaminobenzol, 1,3,5-Triethyl-2,4-diaminobenzol, 3,5,3',5'-Tetraethyl-4,4'-diaminodiphenylmethan, 3,5,3',5'-Tetraisopropyl-4,4'-diaminodiphenylmethan, 3,5-Diethyl-3',5'-düsopropyl-4,4'-diaminodiphenylmethan, 3,3'-Diethyl-5,5'-diisopropyl-4,4'-diaminodiphenylmethan, 1-Methyl-2,6-diamino-3-isopropylbenzol, flüssige Gemische von Polyphenylpolymethylenpolyaminen, wie sie auf bekannte Weise durch Kondensation von Anilin mit Formaldehyd erhältlich sind, sowie beliebige Mischungen solcher Polyamine. In diesem Zusammenhang seien beispielsweise Mischungen von 1-Methyl-3,5-diethyl-2,4-diaminobenzol mit 1-Methyl-3,5-diethyl-2,6-diaminobenzol in einem Gewichtsverhältnis von 50 : 50 bis 85 : 15, vorzugsweise von 65 : 35 bis 80 : 20 besonders erwähnt.

Die Verwendung niedermolekularer aminofunktioneller Polyether mit Molekulargewichten unter 500 g/mol ist ebenfalls möglich. Hierbei handelt es sich beispielsweise um solche mit primären und/oder sekundären, aromatisch oder aliphatisch gebundenen Aminogruppen, deren Aminogruppen gegebenenfalls über Urethan- oder Estergruppen an die Polyetherketten angebunden sind und die nach bekannten, bereits oben zur Herstellung der höhermolekularen Aminopolyether beschriebenen Verfahren zugänglich sind.

Gegebenenfalls können auch sterisch gehinderte aliphatische Diamine mit zwei sekundär gebundenen Aminogruppen als gegenüber Isocyanatgruppen reaktive Komponenten eingesetzt werden, wie z. B. die aus EP-A 0 403 921 bekannten Umsetzungsprodukte aliphatischer und/oder cycloaliphatischer Diamine mit Maleinsäure- oder Fumarsäureestern oder die beispielsweise in der DE-A 19 701 835 beschriebenen Hydrierungsprodukte aus aliphatischen und/oder cycloaliphatischen Diaminen und Ketonen, wie z. B. Diisopropylketon, zugänglicher Schiffscher Basen.

Neben den genannten Ausgangskomponeten A) und B) können dabei gegebenenfalls Hilfs- und Zusatzmittel C), wie z. B. Katalysatoren, oberflächenaktive Mittel, UV-Stabilisatoren, Antioxidantien, Duftstoffe, Formtrennmittel, Füllstoffe und/ oder Pigmente, mitverwendet werden.

Zur Reaktionsbeschleunigung können beispielsweise übliche aus der Polyurethanchemie bekannte Katalysatoren zum Einsatz kommen. Beispielhaft seien hier genannt tert. Amine, wie z. B. Triethylamin, Tributylamin, Dimethylbenzylamin, Diethylbenzylamin, Pyridin, Methylpyridin, Dicyclohexylmethylamin, Dimethylcyclohexylamin, N,N,N',N'-Tetramethyldiaminodiethylether, Bis-(dimethylaminopropyl)-harnstoff, N-Methyl- bzw. N-Ethylmorpholin, N-Cocomorpholin, N-Cyclohexylmorpholin, N,N,N',N'-Tetramethylethylendiamin, N,N,N',N'-Tetramethy-1,3-butandiamin, N,N,N',N'-Tetramethyl-1,6-hexandiamin, Pentamethyldiethylentriamin, N-Methylpiperidin, N-Dimethylaminoethylpiperidin, N,N'-Dimethylpiperazin, N-Methyl-N'-dimethylaminopiperazin, 1,8-Diazabicyclo(5.4.0)undecen-7 (DBU), 1,2-Dimethylimidazol, 2-Methylimidazol, N,N-Dimethylimidazol-β-phenylethylamin, 1,4-Diazabicyclo-(2,2,2)-octan, Bis-(N,N-dimethylaminoethyl)adipat; Alkanolaminverbindungen, wie z. B. Triethanolamin, Triisopropanolamin, N-Methyl- und N-Ethyldiethanolamin, Dimethylaminoethanol, 2-(N,N-Dimethylaminoethoxy)ethanol, N,N',N"-Tris-(dialkylaminoalkyl)hexahydrotriazine, z.B. N, N',N"-Tris-(dimethylaminopropyl)-s-hexahydrotriazin und/oder Bis(dimethylaminoethyl)ether; Metallsalze, wie z. B. anorganische und/oder organische Verbindungen des Eisens, Bleis, Wismuths, Zinks, und/oder Zinns in üblichen Oxidationsstufen des Metalls, beispielsweise Eisen(II)-chlorid, Eisen(III)-chlorid, Wismut(III)- Wismut(III)-2-ethylhexanoat, Wismut(III)-octoat, Wismut(III)-neodecanoat, Zinkchlorid, Zink-2-ethylcaproat, Zinn(II)-octoat, Zinn(II)-ethylcaproat, Zinn(II)-palmitat, Dibutylzinn(IV)-dilaurat (DBTL), Dibutylzinn(IV)-dichlorid, Dimetylzinn(IV)-dichlorid oder Bleioctoat; Amidine, wie z. B. 2,3-Dimethyl-3,4,5,6-tetrahydropyrimidin; Tetraalkylammoniumhydroxide, wie z. B. Tetramethylammoniumhydroxid; Alkalihydroxide, wie z. B. Natriumhydroxid und Alkalialkoholate, wie z. B. Natriummethylat und Kaliumisopropylat, sowie Alkalisalze von langkettigen Fettsäuren mit 10 bis 20 C-Atomen und gegebenenfalls seitenständigen OH-Gruppen.

Bevorzugte einzusetzende Katalysatoren C) sind tertiäre Amine, Bismuth- und Zinnverbindungen der genannten Art.

Die beispielhaft genannten Katalysatoren können bei der Herstellung der erfindungsgemäßen lichtechten Polyurethan-, Polythiourethan- und/oder Polyharnstoffinassen einzeln oder in Form beliebiger Mischungen untereinander eingesetzt werden und kommen dabei gegebenenfalls in Mengen von 0,001 bis 5,0 Gew.-%, bevorzugt 0,002 bis 2 Gew.-%, berechnet als Gesamtmenge an eingesetzten Katalysatoren bezogen auf die Gesamtmenge der verwendeten Ausgangsverbindungen, zum Einsatz.

Aus den erfindungsgemäßen Zusammensetzungen werden vorzugsweise transparente kompakte Formteile mit hohem Brechungsindex hergestellt.

Die aus den erfindungsgemäßen Zusammensetzungen erhaltenen Körper zeichnen sich bereits als solche, d. h. ohne Zusatz entsprechender Stabilisatoren, durch eine sehr gute Lichtbeständigkeit aus. Dennoch können bei ihrer Herstellung gegebenenfalls UV-Schutzmittel (Lichtstabilisatoren) oder Antioxidantien der bekannten Art als Hilfs- und Zusatzmittel C) mitverwendet werden.

Geeignete UV-Stabilisatoren C) sind beispielsweise Piperidinderivate, wie z. B. 4-Benzoyloxy-2,2,6,6-tetramethylpiperidin, 4-Benzoyloxy-1,2,2,6,6-pentamethylpiperidin, Bis-(2,2,6,6-tetramethyl-4-piperidyl)sebacat, Bis(1,2,2,6,6-pentamethyl-4-piperidyl)sebacat, Methyl(1,2,2,6,6-pentamethyl-4-piperidyl)sebacat, Bis-(2,2,6,6-tetramethyl-4-piperidyl)-suberat oder Bis-(2,2,6,6-tetramethyl-4-piperidyl)-dodecandioat, Benzophenonderivate, wie z. B. 2,4-Dihydroxy-, 2-Hydroxy-4-methoxy-, 2-Hydroxy-4-octoxy-, 2-Hydroxy-4-dodecyloxy- oder 2,2'-Dihydroxy-4-dodecyloxy-benzophenon, Benztriazolderivate, wie z. B. 2-(5-Methyl-2-hydroxyphenyl)benztriazol, 2-(5-tert.-Butyl-2-hydroxyphenyl)benztriazol, 2-(5-tert.-Octyl-2-hydroxyphenyl)benztriazol, 2-(5-Dodecyl-2-hydroxyphenyl)benztriazol, 2-(3,5-Di-tert.-butyl-2-hydroxyphenyl)-5-chlorbenztriazol, 2-(3,5-Di-tert.-amyl-2-hydroxyphenyl)benztriazol, 2-(3,5-Di-tert.-butyl-2-hydroxyphenyl)benztriazol, 2-(3-tert.-Butyl-5-methyl-2-hydroxyphenyl)-5-chlorbenztriazol und Veresterungsprodukte von 2-(3-tert.-Butyl-5-propionsäure-2-hydroxyphenyl)benztriazol mit Polyethylenglykol 300, Oxalanilide, wie z. B. 2-Ethyl-2'-ethoxy- oder 4-Methyl-4'-methoxyoxalanilid, Salicylsäureester, wie z. B. Salicylsäurephenylester, Salicylsäure-4-tert.-butylphenylester und Salicylsäure-4-tert.-octylphenylester, Zimtsäureesterderivate, wie z. B. α-Cyano-β-methyl-4-methoxyzimtsäuremethylester, α-Cyano-β-methyl-4-methoxyzimtsäurebutylester, α**-**Cyano-β-phenylzimtsäureethylester und α-Cyano-β-phenylzimtsäureisooctylester, oder Malonesterderivate, wie z. B. 4-Methoxybenzylidenmalonsäuredimethylester, 4-Methoxybenzylidenmalonsäurediethylester und 4-Butoxy-benzylidenmalonsäuredimethylester. Diese Lichtstabilisatoren können sowohl einzeln als auch in beliebigen Kombinationen untereinander zum Einsatz kommen.

Geeignete Antioxidantien C) sind beispielsweise die bekannten sterisch gehinderten Phenole, wie z. B. 2,6-Di-tert-butyl-4-methylphenol (Ionol), Pentaerythrit-tetrakis(3-(3,5-di-tert-butyl-4-hydroxyphenyl)propionat), Octadecyl-3-(3,5-di-tert-butyl-4-hydroxyphenyl)-propionat, Triethylenglykol-bis(3-tert-butyl-4-hydroxy-5-methylphenyl)propionat, 2,2'-Thio-bis(4-methyl-6-tert-butylphenol), 2,2'-Thiodiethyl-bis[3-(3,5-di-tert-butyl-4-hydroxyphenyl)propionat), die sowohl einzeln als auch in beliebigen Kombinationen untereinander zum Einsatz kommen.

Weitere, gegebenenfalls mitzuverwendende Hilfs- und Zusatzmittel C) sind beispielsweise die bekannten Flammschutzmittel, wie z.B. Tris-chlorethylphosphat, Ammoniumphosphat oder -polyphosphat, Füllstoffe, wie z. B. Bariumsulfat, Kieselgur, Russ, Schlämmkreide oder auch verstärkend wirkende Glasfasern.

Schließlich können gegebenenfalls auch die an sich bekannten inneren Formtrennmittel Farbstoffe, Pigmente, Hydrolyseschutzmittel, fungistatische und bakteriostatisch wirkende Substanzen mitverwendet werden.

Besonders bevorzugt enthält die erfindungsgemäße Zusammensetzung als Komponente C) zumindest ein Formtrennmittel, das ausgewählt ist aus Mono- und / oder Dialkylphosphaten und / oder Mono- und / oder Dialkoxyalkylphosphaten. Als Mono- und / oder Dialkylphosphate sind insbesondere Mono- und / oder Dialkylphosphate mit 2 bis 18 Kohlenstoffatomen im Alkylrest, bevorzugt 8 bis 12 Kohlenstoffatomen. Besonders bevorzugte Mono- beziehungsweise Dialkoxyalkylphosphate besitzen 2 bis 12 Kohlenstoffatome im Alkoxyalkylrest und bis zu drei Ethergruppen pro Alkoxyalkylrest, wobei die vorgenannten Mono- beziehungsweise Alkoxyalkylphosphate insbesondere 4 bis 10 Kohlenstoffatome im Alkoxyalkylrest aufweisen. Die bevorzugten Mono- / Dialkylphosphate und Mono- / Dialkoxyalkylphosphate sind besonders vorteilhaft, da sie neben ihrer eigentlichen Funktion als Formtrennmittel zusätzlich noch einen günstigen Einfluss auf die Reaktionsgeschwindigkeit bei der Umsetzung der Polyisocyanatkomponente mit der Thiolkomponente insofern zeigen, dass durch deren Zusatz die Reaktionsgeschwindigkeit der beiden Komponenten miteinander reduziert wird. Dies führt letztlich zu optisch hochwertigeren Formkörpern und einer einfacheren Handhabung der Zusammensetzung nach dem Vermischen der Komponenten.

Geeignete Formtrennmittel sind beispielsweise Methylphosphat, Dimethylphosphat, Methoxyethylphosphat, Methoxypropylphosphat, Di-(methoxyethyl)phosphat, Methoxyethyl-ethoxyethylphosphat, Methoxyethyl-propoxyethylphosphat, Di-(methoxypropyl)phosphat, Ethylphosphat, Diethylphosphat, Ethoxyethylphosphat, Di-(ethoxyethyl)phosphat, Ethoxypropylphosphat, Ethoxyethyl-propoxyethylphosphat, Di-(ethoxypropyl)phosphat, Ethoxyethyl-butoxyethylphosphat, Isopropylphosphat, Diisopropylphosphat, Propoxyethylphosphat, Di-(propoxyethyl)phosphat, Propoxypropylphosphat, Di-(propoxylpropyl)phosphate, Butylphosphat, Dibutylphosphat, Butoxyethylphosphat, Butoxypropylphosphat, Di-(butoxyethyl) phosphat, Pentoxyethylphosphat, Bis-(2-ethylhexyl)phosphat, Di-(hexyloxyethyl)phosphat, Octylphosphat, Dioctylphosphat, Decylphosphat, Isodecylphosphat, Diisodecylphosphat, Isodecyloxyethylphosphat, Di-(decyloxyethyl)phosphat, Dodecylphosphat, Didoceylphosphat, Tridecanolephosphat, Bis(tridecanol)-phosphat, Stearylphospat, Distearylphosphat und beliebige Gemische solcher Verbindungen.

Zweckmäßigerweise enthalten die erfindungsgemäßen Zusammensetzungen bezogen auf die Gesamtzusammensetzung 0,01 bis 4 Gew.-% an Mono- / Dialkylphosphaten und / oder Mono- / Dialkylalkoxyphosphaten, bevorzugt 0,02 bis 2 Gew.-%. Die vorgenannten Einsatzmengen beziehen sich dabei auf den Gesamtgehalt dieser Substanzen als Formtrennmittel.

Eine besonders bevorzugte erfindungsgemäße Zusammensetzung zur Herstellung transparenter Polythiourethankörper enthält oder besteht aus
A) 35 bis 65 Gew.-% bezogen auf die Zusammensetzung, insbesondere 45 bis 55 Gew.-% einer Polyisocyanatkomponente enthaltend
   zumindest 90 bis 99,8 Gew.-%, insbesondere 95 bis 99,7 Gew.-% bezogen auf die Polyisocyanatkomponente eines Polyisocyanat ausgewählt aus 1,3-XDI und/ oder 1,4-XDI mit einer Funktionalität an Isocyanatgruppen von 2 pro Molekül und 0,05 bis 2 Gew.-%, insbesondere 0,1 bis 1 Gew.-% bezogen auf die Polyisocyanatkomponente an 3-(Isocyanatomethyl)benzonitril und/oder 4-(Isocyanatomethyl)benzonitril,
B) 35 bis 65 Gew.-% bezogen auf die Zusammensetzung, insbesondere 45 bis 55 Gew.-% einer Thiolkomponente enthaltend zumindest ein Polythiol mit einer Funktionalität an Thiolgruppen von wenigstens 2 pro Molekül, wobei die Thiolkomponente insbesondere aus einem Polythiol mit einer Funktionalität an Thiolgruppen von 3 pro Molekül, nämlich DMPT (4-Mercaptomethyl-1,8-dimercapto-3,6-dithiaoctan), besteht,
   sowie
C) Hilfs- und Zusatzmittel umfassend 0,01 bis 4 Gew.-%, insbesondere 0,02 bis 2 Gew.-% eines Formtrennmittels ausgewählt aus Mono- und/ oder Dialkylphosphaten mit 2 bis 18 Kohlenstoffatomen im Alkylrest und/oder Mono- und/ oder Dialkoxyalkylphosphaten mit 2 bis 12 Kohlenstoffatomen im Alkoxyalkylrest und bis zu drei Ethergruppen pro Alkoxyalkylrest,
   wobei das Verhältnis von Isocyanatgruppen zu gegenüber Isocyanaten reaktiven Gruppen 0,5 : 1 bis 2,0 : 1 beträgt,
   wobei die Zusammensetzung dadurch gekennzeichnet ist, dass
   die Zusammensetzung ferner 0,005 bis 10 Gew.-% bezogen auf die gesamte Zusammensetzung als Komponente D) zumindest eines aromatischen Nitrils enthält, das ausgewählt ist aus 3-(Isocyanatomethyl)benzonitril, und/ oder 4-(Isocyanatomethyl)benzonitril, insbesondere 0,01 bis 5 Gew.-%.

Ein weiterer Gegenstand der vorliegenden Erfindung betrifft ein Verfahren zur Herstellung transparenter Polythiourethankörper durch Umsetzung einer Zusammensetzung enthaltend oder bestehend aus
A) einer Polyisocyanatkomponente enthaltend zumindest ein Polyisocyanat mit einer Funktionalität an Isocyanatgruppen von wenigstens 2 pro Molekül,
B) einer Thiolkomponente enthaltend oder bestehend aus zumindest ein Polythiol mit einer Funktionalität an Thiolgruppen von wenigstens 2 pro Molekül, sowie gegebenenfalls
C) Hilfs- und Zusatzmittel,
   wobei das Verhältnis von Isocyanatgruppen zu gegenüber Isocyanaten reaktiven Gruppen 0,5 : 1 bis 2,0 : 1 beträgt,
   wobei das das Verfahren dadurch gekennzeichnet ist, dass die Zusammensetzung ferner
D) zumindest ein aromatisches Nitril enthält.

Für die im erfindungsgemäßen Verfahren eingesetzten Zusammensetzungen gelten analog die oben ausgeführten bevorzugten Ausführungsformen und Definitionen.

Das Verfahren kann insbesondere ohne Lösemittelzusatz, also lösemittelfrei, durchgeführt werden, wobei die Nitrilzugabe in diesem Zusammenhang nicht als Lösemittelzugabe zu verstehen ist.

Unabhängig von der Art der gewählten Ausgangsstoffe erfolgt beim erfindungsgemäßen Verfahren die Umsetzung der Polyisocyanatgemische A) mit der Thiolkomponente B) sowie gegebenenfalls weiteren gegenüber Isocyanatgruppen reaktiven Komponenten unter Einhaltung eines Äquivalentverhältnisses von Isocyanatgruppen zu gegenüber Isocyanaten reaktiven Gruppen von 0,5 : 1 bis 2,0 : 1, vorzugsweise von 0,7 : 1 bis 1,3 : 1, besonders bevorzugt von 0,8 : 1 bis 1,2 : 1.

Bei dem erfindungsgemäßen Verfahren werden die Komponenten der erfindungsgemäßen Zusammensetzung bevorzugt, gegebenenfalls in lösemittelfreier Form, im oben angegebenen Äquivalentverhältnis von Isocyanatgruppen zu gegenüber Isocyanaten reaktiven Gruppen, mit Hilfe geeigneter Mischaggregate gemischt und nach beliebigen Methoden in offene oder geschlossene Formen, beispielsweise durch einfaches Vergießen von Hand, bevorzugt aber mit Hilfe geeigneter Maschinen, wie z. B. den in der Polyurethantechnologie üblichen Niederdruck- oder Hochdruckmaschinen oder nach dem RIM-Verfahren, eingefüllt. Die Aushärtung kann in einem Temperaturbereich von 40 bis 180°C, vorzugsweise von 50 bis 140°C, besonders bevorzugt von 60 bis 120°C, und gegebenenfalls unter einem erhöhten Druck von bis zu 300 bar, vorzugsweise bis 100 bar, besonders bevorzugt bis zu 40 bar durchgeführt werden.

Dabei können die Polyisocyanate und gegebenenfalls auch die anderen Ausgangskomponenten durch Anlegen von Vakuum entgast werden.

In der Regel können die so aus den erfindungsgemäß hergestellten Formkörper nach kurzer Zeit entformt werden, beispielsweise nach 2 bis 60 min. Gegebenenfalls kann sich eine Nachhärtung bei einer Temperatur von 50 bis 100 °C anschließen, vorzugsweise bei 60 bis 90°C.

Auf diese Weise erhält man kompakte licht- und wetterbeständige Polythiourethankörper, die eine hohe Beständigkeit gegenüber Lösungsmitteln und Chemikalien sowie hervorragende mechanische Eigenschaften, insbesondere eine exzellente Wärmeformbeständigkeit auch bei höheren Temperaturen von beispielsweise 80°C, besitzen. Gegenüber den bisher bekannten Systemen, die keine aromatischen Nitrile enthalten, zeichnen sich die erfindungsgemäßen Formkörper durch eine geringere Neigung zur Bildung von Trübung aus.

Ein weiterer Gegenstand der vorliegenden Erfindung betrifft einen kompakten transparenten Polythiourethankörper, erhältlich durch Umsetzung der Komponenten der erfindungsgemäßen Zusammensetzung. Bei dem Polythiourethankörper kann es sich hierbei um ein Glasersatzteil, ein optisches, optoelektronisches oder elektronisches Bauteil, um eine optische Linse oder um ein Brillenglas handeln. Konkrete Anwendungen sind beispielsweise die Herstellung von bzw. die Verwendung als Glasersatz-Scheiben, wie z. B. Sonnendächern, Front-, Heck- oder Seitenscheiben im Fahrzeug- oder Flugzeugbau, als Sicherheitsglas, Solarmodulen, Leuchtdioden, Linsen oder Kollimatoren, wie sie beispielsweise als Vorsatzoptik in LED-Leuchten oder Automobilscheinwerfern zum Einsatz kommen.

Besonders bevorzugtes Einsatzgebiet für die aus den erfindungsgemäßen Zusammensetzungen erhältlichen erfindungsgemäßen Polythiourethan-Formkörpern ist aber die Herstellung leichtgewichtiger Kunststoff-Brillengläser mit hohem Brechungsindex und hoher Abbe-Zahl. Erfindungsgemäß hergestellte Brillengläser zeichnen sich durch hervorragende mechanische Eigenschaften, insbesondere Härte und Schlagfestigkeit sowie gute Kratzfestigkeit aus und sind darüber hinaus leicht zu bearbeiten und beliebig einfärbbar. Die vorliegende Erfindung betrifft ferner die Verwendung von aromatischen Nitrilen zur Herstellung transparenter Polythiourethankörper. Offenbart wird ferner eine Mischung aus einem Polyisocyanat mit einer Funktionalität an Isocyanatgruppen von wenigstens 2 pro Molekül und wenigstens einem aromatischen Nitril zur Herstellung kompakter transparenter Polythiourethankörper.

Die Erfindung wird im Folgenden anhand von Ausführungsbeispielen näher erörtert.

### Beispiele

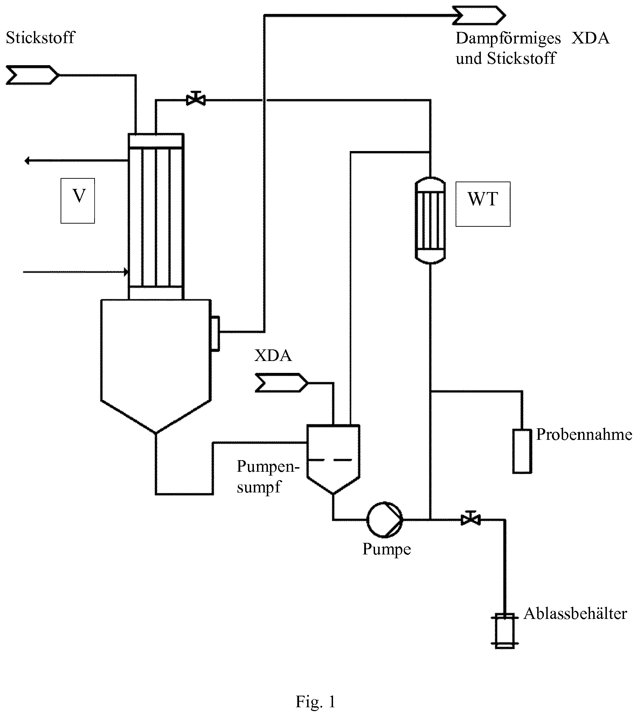

In Fig. 1 ist eine geeignete Anlage zur Gasphasenphosgenierung schematisch abgebildet. Diese Anlage eignet sich insbesondere zur Erzeugung von 1,3-XDI und 1,4-XDI mit Gehalten an Nitrilen >0,1 Gew.-% bezogen auf das erzeugte Isocyanat.

Alle Prozentangaben beziehen sich, soweit nichts Anderslautendes vermerkt, auf das Gewicht.

Die Bestimmung der NCO-Gehalte erfolgte titrimetrisch nach DIN EN ISO 11909.

Die Messung der Brechungsindices und Abbe-Zahlen erfolgte an einem Abbe-Refraktometer Modell B der Fa. Zeiss nach DIN EN ISO 489:1999-08.

Die Transmissions- und Haze- Messungen nach ASTM D 1003 wurden mit einem Haze-Gard Plus der Fa. Byk bei Normlichtart D65 (definiert in DIN 6173) durchgeführt.

Die verwendeten Chemikalien wurden ohne weitere Vorbehandlung eingesetzt:
Tinuvin ® 571: Alkylphenol-substituiertes Benztriazol (BASF)
Zelec UN: Mischung aus langkettigen Mono- und Dialkylphosphat (Steppan)
DBC: Dibutylzinndichlorid (Acros Organics)
DMPT: 4-Mercaptomethyl-1,8-dimercapto-3,6-dithiaoctan (Bruno Bock GmbH)
Desmodur W: H12-MDI, NCO Funktionalität 2 (Bayer MaterialScience AG) Benzonitril (Merck KGaA)

### Durchführung der Gasphasen Phosgenierung von 1,3-XDA

### Beispiel 1:

In einer Anlage zur Gasphasenphosgenierung mit einer Aminverdampfungsstufe entsprechend Fig. 1, einem Rohreaktor (L: 9350 mm, Innendurchmesser 134,5 mm) mit einer auf der Reaktorachse angeordneten Koaxialdüse (Innendurchmesser 134,5 mm) und einer nachgeschalteten Isocyanatkondensationsstufe wurden kontinuierlich 200 kg/h 1,3-XDA bei einem Druck von 650 mbar abs. unter Einleitung eines Stickstoffstroms von 10 kg/h verdampft, wobei die Temperatur im Umpumpkreislauf (3200 kg/h) durch Kühlung in einem Wärmetauscher (WT) bei 150°C gehalten wurde. Dem Umpumpkreislauf wurden vorher 500 ppm Tinuvin ® 571 zugesetzt. Die Vorlauftemperatur zum Verdampfer (V) betrug 255°C, die Eintrittstemperatur des Kühlmediums in den Wärmetauscher (WT) 40°C und die mittlere Verweilzeit des 1,3-XDA im Umpumpkreislauf 35 Minuten. Der Strom aus gasförmigem 1,3-XDA und Stickstoff wurde nach Verlassen des Verdampfers in einem weiteren Wärmetauscher auf 280°C erhitzt und über die Koaxialdüse dem Reaktor zugeführt. Gleichzeitig wurden parallel dazu 750 kg/h Phosgen auf 310°C erhitzt und auf dem von der Düse freigelassenen Ringraum ebenfalls dem Reaktor kontinuierlich zugeführt, in welchem die beiden Eduktströme gemischt und zur Reaktion gebracht wurden. Dabei betrug die Geschwindigkeit des Gasstroms im Reaktor ca. 20 m/s und das Geschwindigkeitsverhältnis von Amin-/Stickstoff- zu Phosgenstrom 8,8. Der Druck an der Vakuumpumpe lag bei 600 mbar abs.

Nach einer mittleren Verweilzeit im Reaktor von 0,48 s wurde der das Reaktionsprodukt 1,3-XDI enthaltende Gasstrom durch Einspritzkühlung mit Monochlorbenzol abgekühlt und kondensiert, wobei die Temperatur der flüssigen Phase in der Quenche ca. 90°C betrug. Der gaschromatographisch bestimmte Gehalt an 3-Chlormethyl-benzylisocyanat betrug 0,4 % bezogen auf die Summe aus 1,3-XDI und 3-Cl-XI. Nachfolgend wurde das Reaktionsgemisch von HCl und Phosgen befreit und destillativ aufgearbeitet. Die Ausbeute an 1,3-XDI betrug 95% der Theorie.

### Beispiel 2:

In der oben beschriebenen Anlage wurden analog 160 kg/h 1,3-XDA bei einem Druck von 500 mbar abs. unter Einleitung eines Stickstoffstroms von 4 kg/h verdampft, wobei die Temperatur im Umpumpkreislauf (3200 kg/h) durch Kühlung in einem Wärmetauscher (WT) bei 150°C gehalten wurde. Dem Umpumpkreislauf wurden vorher 500 ppm Tinuvin ® 571 zugesetzt. Die Vorlauftemperatur zum Verdampfer (V) betrug 240°C, die Eintrittstemperatur des Kühlmediums in den Wärmetauscher (WT) 40°C und die mittlere Verweilzeit des 1,3-XDA im Umpumpkreislauf 43 Minuten. Der Strom aus gasförmigem1,3-XDA und Stickstoff wurde in einem weiteren Wärmetauscher auf 280°C erhitzt und über die Koaxialdüse dem Reaktor zugeführt. Gleichzeitig wurden parallel dazu 500 kg/h Phosgen auf 310°C erhitzt und auf dem von der Düse freigelassenen Ringraum ebenfalls dem Reaktor kontinuierlich zugeführt, in welchem die beiden Eduktströme gemischt und zur Reaktion gebracht wurden. Dabei betrug die Geschwindigkeit des Gasstroms im Reaktor ca. 20 m/s und das Geschwindigkeitsverhältnis von Amin- zu Phosgenstrom 8,8. Nach einer mittleren Verweilzeit im Reaktor von 0,46 s wurde der das Reaktionsprodukt 1,3-XDI enthaltende Gasstrom durch Einspritzkühlung mit Monochlorbenzol abgekühlt und kondensiert, wobei die Temperatur der flüssigen Phase in der Quenche ca. 90°C betrug.

Der gaschromatographisch bestimmte Gehalt an 3-Chlormethyl-benzylisocyanat betrug 0,3 % bezogen auf die Summe aus 1,3-XDI und 1,3-Cl-XI. Nachfolgend wurde das Reaktionsgemisch von HCl und Phosgen befreit und destillativ aufgearbeitet. Die Ausbeute an 1,3-XDI betrug 92% der Theorie.

### Herstellung von über Flüssigphasen-Phosgenierung erzeugtem 1,3-XDI

### Beispiel 3:

Unter Rühren und Kühlen wurde eine Lösung von 5 Gew.-Teilen 1,3-XDA in 50 Gew.-Teilen Monochlorbenzol bei 0 - 10°C zu einer Lösung von 20 Gew.-Teilen Phosgen in 25 Gew.-Teilen Monochlorbenzol dosiert und nach beendeter Zugabe auf Raumtemperatur kommen gelassen. Anschließend wurde die Temperatur unter Einleiten von Phosgen entsprechend der Gasentwicklung bis zum Rückfluss erhöht und solange weiter phosgeniert, bis die Lösung allmählich klar wurde. Sobald der Klarpunkt erreicht war (ca. 4-5 h), wurde noch 30 Minuten nachphosgeniert. Dann wurde die Phosgeneinleitung beendet und unter Einleitung von Stickstoff solange am Rückfluss gekocht, bis im Abgas kein Phosgen mehr nachweisbar war.

Anschließend wurde das Reaktionsgemisch destillativ aufgearbeitet, wobei XDI als farblose Flüssigkeit mit einem Siedepunkt von 130°C / 0,2mbar erhalten wurde. Die Ausbeute an 1,3-XDI betrug ca. 80% der Theorie.

### Beispiel 4:

2 kg des in Beispiel 2 erhaltenen XDI wurden über eine Kolonne fraktioniert destilliert. Die ersten 500 g wurden als Vorlauf verworfen, als Hauptfraktion wurden 1 kg farbloses 1,3-XDI erhalten. Diese wurden mit 1,4g Zelec UN (Steppan) bei Raumtemperatur versetzt und 24 h stehen gelassen. Der HC Gehalt der Probe, gemessen nach ASTM Vorschrift D4663-98, lag nach der Destillation bei 105 ppm. Zur Bestimmung des Gehalts an 3-Isocyanatomethylbenzonitril wurden 1 g 1,3-XDI in 100 ml Acetonitril gelöst. 100 µl dieser Lösung wurden mit 900 µl einer Diethylamin Lösung (0,2 g Diethylamin in 100 ml Acetonitril) gemischt und 30 min bei 65 °C vor der HPLC-MS Messung gelagert. Die Reinheit wurde bestimmt durch Integration der Flächen der Signale im UV Spektrometer. Dabei wurde angenommen, dass alle Verbindungen die gleiche UV Absorption zeigen und keine Verbindungen ohne UV Absorption in den Proben enthalten sind. Eine weitere Annahme war, dass keinerlei Abbaureaktionen während der Messung stattfinden. Für die HPLC-MS Messung wurde folgendes Programm gewählt:
Synapt G2-S HR-MS, ACQUITY UPLC (Waters) QS. Nr.: 02634
UV: PDA (Total Absorbance Chromatogram)
Säule: Kinetex 100 x 2.1mm_1.7µm
Säulentemperatur: 30 °C

Die mobile Phase bestand aus:
Lösemittel:
   A) Wasser + 0.05% Ameisensäure
   B) Acetonitril + 0.05% Ameisensäure
Flussrate: 0.5 ml/min
Gradient: t0/5%B_t0.5/5%B_t6/100%B_t7/100%B_t7.1/5%B_t8/5%B

In der Probe wurden 98,4 % 1,3-XDI sowie 0,7 % 3-Isocyanatomethylbenzonitril gefunden.

### Beispiel 5:

2 kg des in Beispiel 3 erhaltenen XDI wurden über eine Kolonne fraktioniert destilliert. Die ersten 800 g wurden als Vorlauf verworfen, als Hauptfraktion wurden 700 g farbloses 1,3-XDI erhalten. Diese wurden mit 0,98 g Zelec UN bei Raumtemperatur versetzt und 24 h stehen gelassen. Der HC Gehalt der Probe, gemessen nach ASTM Vorschrift D4663-98, lag nach der Destillation bei 108 ppm. Analog zu Beispiel 4 wurde eine Reinheitsbestimmung per HPLC durchgeführt. Hierbei wurde kein 3-Isocyanatomethylbenzonitril gefunden. Die Anteil an 1,3-XDI lag bei 95,4 %.

### Herstellung von Polythiourethankörpern:

### Beispiel 6:

In einem Kolben wurden in 94,59 g 1,3-XDI aus Beispiel 4 0,002 g Dibutylzinndichlorid (DBC) gelöst und für 30 Minuten bei 10 mbar evakuiert. 90,00 g DMPT (4-Mercaptomethyl-1,8-dimercapto-3,6-dithiaoctan) wurden anschließend in den Kolben dazugegeben, und die finale Mischung wurde für 30 Minuten bei 10 mbar gerührt und entgast. Die Mischung wurde dann durch einen 5 µm Filter filtriert, auf eine Spritze aufgezogen und die Gießform damit vollständig gefüllt. Die Gießform wurde erstellt, indem zwei Glasformschalen (85mm Durchmesser, Innenradius 88 mm, Shamir Insight, Inc., IL) im Abstand von 8 mm mit einem Kunststoff Dichtring zusammengeklemmt wurden, so dass eine Gießkavität gebildet wird. Der Formabstand beträgt an jeder Stelle der Linse 8 mm.

Die gefüllte Gießform wurde im Trockenschrank mit folgendem Temperaturprofil gehärtet: 15 Stunden bei 65 °C; 2 Stunden bei 100 °C und weitere 2 Stunden bei 120 °C getempert. Danach wurde die Gießform auf Raumtemperatur abgekühlt und nach vollständigem Abkühlen zunächst die Manschette und dann die zwei Glaskörper manuell entfernt.

Auf diese Weise wurde ein völlig klarer, transparenter und trübungsfreier Brillenglasrohling erhalten.

Die Transmission betrug bei der Normlichtart D65 90,3 %, der Haze lag bei 2,1. Der Brechungsindex nE betrug 1,67 bei 23 °C.

### Beispiel 7:

Analog zu Beispiel 6 wurde ein Brillenglasrohling hergestellt unter Verwendung von 1,3-XDI aus Beispiel 5. Dieser Brillenglasrohling war vollkommen trübe, die Transmission lag bei lediglich 29,7 %, der Haze bei 100.

### Beispiel 8:

In einem Kolben wurden 94,59 g 1,3-XDI aus Beispiel 5 mit 9,25 g Benzonitril versetzt, darin 0,002 g Dibutylzinndichlorid (DBC) gelöst und für 30 Minuten bei 10 mbar evakuiert. 90,00 g DMPT (4-Mercaptomethyl-1,8-dimercapto-3,6-dithiaoctan) wurden anschließend in den Kolben dazugegeben, und die finale Mischung wurde für 30 Minuten bei 10 mbar gerührt und entgast. Die Mischung wurde dann anschließend wie in Beispiel 6 weiter zu einem Brillenglasrohling weiterverarbeitet.

Auf diese Weise wurde ein völlig klarer, transparenter und trübungsfreier Brillenglasrohling erhalten.

Die Transmission betrug bei der Normlichtart D65 86,3 %, der Haze lag bei 2,9. Der Brechungsindex nE betrug 1,67 bei 23 °C.

### Beispiel 9:

In einem Kolben wurden in 131,00 g Desmodur W, 10,89 g Benzonitril und 1,31 g Zelec UN 0,011 g Dibutylzinndichlorid (DBC) gelöst und für 30 Minuten bei 10 mbar evakuiert. 85,50 g DMPT (4-Mercaptomethyl-1,8-dimercapto-3,6-dithiaoctan) wurden anschließend in den Kolben dazugegeben, und die finale Mischung wurde für 30 Minuten bei 10 mbar gerührt und entgast. 20 g der Mischung wurden dann in einen verschließbaren PP-Becher (Salbenkruke 75 g, Innendurchmesser 50 mm) gegeben und im Trockenschrank mit folgendem Temperaturprofil gehärtet: 8 Stunden bei 65 °C; 2 Stunden bei 100 °C und weitere 6 Stunden bei 120 °C getempert. Danach wurde die Gießform auf Raumtemperatur abgekühlt und nach vollständigem Abkühlen der Gießkörper entformt. Der Gießkörper war trübungsfrei und transparent.

### Beispiel 10:

In einem Kolben wurden in 131,00 g Desmodur W und 2,6 g Zelec UN 0,22 g Dibutylzinndichlorid (DBC) gelöst und für 30 Minuten bei 10 mbar evakuiert. 85,50 g DMPT (4-Mercaptomethyl-1,8-dimercapto-3,6-dithiaoctan) wurden anschließend in den Kolben dazugegeben, und die finale Mischung wurde für 30 Minuten bei 10 mbar gerührt und entgast. 20 g der Mischung wurden dann in einen verschließbaren PP-Becher (Salbenkruke 75 g, Innendurchmesser 50 mm) gegeben und im Trockenschrank mit folgendem Temperaturprofil gehärtet: 8 Stunden bei 65 °C; 2 Stunden bei 100 °C und weitere 6 Stunden bei 120 °C getempert. Danach wurde die Gießform auf Raumtemperatur abgekühlt und nach vollständigem Abkühlen der Gießkörper entformt. Der Gießkörper war milchig weiß.

## Patentansprüche

1. Zusammensetzung zur Herstellung transparenter Polythiourethankörper enthaltend oder bestehend aus
A) einer Polyisocyanatkomponente enthaltend zumindest ein Polyisocyanat mit einer Funktionalität an Isocyanatgruppen von wenigstens 2 pro Molekül,
B) einer Thiolkomponente enthaltend zumindest ein Polythiol mit einer Funktionalität an Thiolgruppen von wenigstens 2 pro Molekül,
sowie gegebenenfalls
C) Hilfs- und Zusatzmittel,
wobei das Verhältnis von Isocyanatgruppen zu gegenüber Isocyanaten reaktiven Gruppen 0,5 : 1 bis 2,0 : 1 beträgt,
**dadurch gekennzeichnet, dass** die Zusammensetzung ferner
D) zumindest ein aromatisches Nitril enthält.

2. Zusammensetzung nach Anspruch 1, **dadurch gekennzeichnet, dass** die Zusammensetzung 0,0025 bis 10 Gew.-% bezogen auf die gesamte Zusammensetzung an aromatischem Nitril enthält, insbesondere 0,005 bis 5 Gew.-%.

3. Zusammensetzung nach Anspruch 1 oder 2, **dadurch gekennzeichnet, dass** das Nitril mindestens eine weitere funktionelle Gruppe enthält, insbesondere eine Isocyanat-Gruppe.

4. Zusammensetzung nach einem der vorstehenden Ansprüche, **dadurch gekennzeichnet, dass** das Nitril ausgewählt ist aus Benzonitril, 3-(Isocyanatomethyl)benzonitril, 4-(Isocyanatomethyl)benzonitril, 3-/Chloromethyl)benzonitril, 4-/Chloromethyl)benzonitril oder Mischungen von diesen, insbesondere Benzonitril, 3-(Isocyanatomethyl)benzonitril, und/ oder 4-/Isocyanatomethyl)benzonitril.

5. Zusammensetzung nach einem der vorstehenden Ansprüche, dadurch gekenn-zeichnet, dass das Polyisocyanat ausgewählt ist aus 1,3-Bis(isocyanatomethyl)benzol (1,3 XDI), 1,4-Bis/isocyanatomethyl)benzol (1,4 XDI), 2,5-Bis/isocyanatomethyl)bicyclo[2.2.1]heptan, 2,6-Bis(isocyanatomethyl)bicyclo[2.2.1]heptan, 1,4-Bis/isocyanatomethyl)cyclohexan, 1,3-Bis(isocyanatomethyl)cyclohexan, 2,2'-Diisocyanatodicyclohexylmethan, 2,4'-Diisocyanatodicyclohexylmethan, 4,4'-Diisocyanatodicyclohexylmethan (H12 MDI), 1-Isocyanato-3,3,5-trimethyl-5-isocyanatomethylcyclohexan (Isophorondiisocyanat; IPDI) oder Mischungen von diesen, insbesondere bevorzugt 1,3-Bis(isocyanatomethyl)benzol (1,3 XDI) und/ oder 1,4-Bis(isocyanatomethyl)benzol (1,4 XDI).

6. Zusammensetzung nach einem der vorstehenden Ansprüche, **dadurch gekennzeichnet, dass** das Polyisocyanat ein aromatisches Polyisocyanat ist und das Nitril von demselben Polyamin abgeleitet ist wie das Polyisocyanat.

7. Zusammensetzung nach Anspruch 5 oder 6, **dadurch gekennzeichnet, dass** das Polyisocyanat ausgewählt ist aus 1,3-Bis(isocyanatomethyl)benzol (1,3 XDI) und das Nitril aus 3-(Isocyanatomethyl)benzonitril und/oder dass das Polyisocyanat ausgewählt ist aus 1,4-Bis(isocyanatomethyl)benzol (1,4 XDI) und das Nitril aus 4-(Isocyanatomethyl)benzonitril.

8. Zusammensetzung nach einem der vorstehenden Ansprüche, **dadurch gekennzeichnet, dass** das Polythiol ausgewählt ist aus 4-Mercaptomethyl-1,8-dimercapto-3,6-dithiaoctan, 2,5-Bismercaptomethyl-1,4-dithian, 1,1,3,3-Tetrakis/mercaptomethylthio)propan, 5,7-Dimercaptomethyl-1,11-dimercapto-3,6,9-trithiaundecan, 4,7-Dimercaptomethyl-1,11-dimercapto-3,6,9-trithiaundecan, 4,8-Dimercaptomethyl-1,11-dimercapto-3,6,9-trithiaundecan, Trimethylolpropan-tris(3-mercaptopropionat), Trimethylolethan-tris(2-mercaptoacetat), Pentaerythrit-tetrakis(2-mercaptoacetat) und/ oder Pentaerythrittetrakis(3-mercaptopropionat).

9. Zusammensetzung nach einem der vorstehenden Ansprüche, **dadurch gekennzeichnet, dass** als Komponente C) Katalysatoren, oberflächenaktive Mittel, UV-Stabilisatoren, Antioxidantien, Duftstoffe, Formtrennmittel, Füllstoffe und/ oder Pigmente eingesetzt werden.

10. Zusammensetzung nach Anspruch 9, **dadurch gekennzeichnet, dass** als Formtrennmittel Phosphorsäureester eingesetzt werden, insbesondere Mono- und/ oder Dialkylphosphate mit 2 bis 18 Kohlenstoffatomen im Alkylrest, bevorzugt mit 8 bis 12 Kohlenstoffatomen.

11. Zusammensetzung nach Anspruch 9 oder 10, **dadurch gekennzeichnet, dass** als Formtrennmittel Mono- und/ oder Dialkoxyalkylphosphate mit 2 bis 12 Kohlenstoffatomen im Alkoxyalkylrest und bis zu drei Ethergruppen pro Alkoxyalkylrest, insbesondere 4 bis 10 Kohlenstoffatomen.

12. Verfahren zur Herstellung transparenter Polythiourethankörper durch Umsetzung einer Zusammensetzung enthaltend oder bestehend aus
A) einer Polyisocyanatkomponente enthaltend zumindest ein Polyisocyanat mit einer Funktionalität an Isocyanatgruppen von wenigstens 2 pro Molekül,
B) einer Thiolkomponente enthaltend zumindest ein Polythiol mit einer Funktionalität an Thiolgruppen von wenigstens 2 pro Molekül,
sowie gegebenenfalls
C) Hilfs- und Zusatzmittel,
wobei das Verhältnis von Isocyanatgruppen zu gegenüber Isocyanaten reaktiven Gruppen 0,5 : 1 bis 2,0 : 1 beträgt,
**dadurch gekennzeichnet, dass** die Zusammensetzung ferner
D) zumindest ein aromatisches Nitril enthält.

13. Kompakter transparenter Polythiourethankörper erhältlich durch Umsetzung der Komponenten einer Zusammensetzung nach einem der Ansprüche 1 bis 11, wobei es sich bei dem Polythiourethankörper insbesondere um ein Glasersatzteil, ein optisches, optoelektronisches oder elektronisches Bauteil, um optische Linsen oder Brillengläser handelt.

14. Verwendung von aromatischen Nitrilen zur Herstellung transparenter Polythiourethankörper.

## Claims

1. Composition for producing transparent polythiourethane articles containing or consisting of
A) a polyisocyanate component containing at least one polyisocyanate having a functionality of isocyanate groups of at least 2 per molecule,
B) a thiol component containing at least one polythiol having a functionality of thiol groups of at least 2 per molecule
and optionally
C) auxiliary and additive agents,
wherein the ratio of isocyanate groups to isocyanate-reactive groups is 0.5 : 1 to 2.0 : 1, **characterized in that** the composition further contains
D) at least one aromatic nitrile.

2. Composition according to Claim 1, **characterized in that** the composition contains 0.0025 to 10 wt% based on the entire composition of aromatic nitrile, in particular 0.005 to 5 wt%.

3. Composition according to Claim 1 or 2, **characterized in that** the nitrile contains at least one further functional group, in particular an isocyanate group.

4. Composition according to any of the preceding claims, **characterized in that** the nitrile is selected from benzonitrile, 3-(isocyanatomethyl)benzonitrile, 4-(isocyanatomethyl)benzonitrile, 3-(chloromethyl)benzonitrile, 4-(chloromethyl)benzonitrile or mixtures thereof, in particular benzonitrile, 3-(isocyanatomethyl)benzonitrile, and/or 4-(isocyanatomethyl)benzonitrile.

5. Composition according to any of the preceding claims, **characterized in that** the polyisocyanate is selected from 1,3-bis(isocyanatomethyl)benzene (1,3-XDI), 1,4-bis(isocyanatomethyl)benzene (1,4-XDI), 2,5-bis(isocyanatomethyl)bicyclo[2.2.1]heptane, 2,6-bis(isocyanatomethyl)bicyclo[2.2.1]heptane, 1,4-bis(isocyanatomethyl)cyclohexane, 1,3-bis(isocyanatomethyl)cyclohexane, 2,2'-diisocyanatodicyclohexylmethane, 2,4'-diisocyanatodicyclohexylmethane, 4,4'-diisocyanatodicyclohexylmethane (H12-MDI), 1-isocyanato-3,3,5-trimethyl-5-isocyanatomethylcyclohexane (isophorone diisocyanate; IPDI) or mixtures thereof, especially preferably 1,3-bis(isocyanatomethyl)benzene (1,3-XDI) and/or 1,4-bis(isocyanatomethyl)benzene (1,4-XDI).

6. Composition according to any of the preceding claims, **characterized in that** the polyisocyanate is an aromatic polyisocyanate and the nitrile is derived from the same polyamine as the polyisocyanate.

7. Composition according to Claim 5 or 6, **characterized in that** the polyisocyanate is selected from 1,3-bis(isocyanatomethyl)benzene (1,3-XDI) and the nitrile from 3-(isocyanatomethyl)benzonitrile and/or that the polyisocyanate is selected from 1,4-bis(isocyanatomethyl)benzene (1,4-XDI) and the nitrile from 4-(isocyanatomethyl)benzonitrile.

8. Composition according to any of the preceding claims, **characterized in that** the polythiol is selected from 4-mercaptomethyl-1,8-dimercapto-3,6-dithiaoctane, 2,5-bismercaptomethyl-1,4-dithiane, 1,1,3,3-tetrakis(mercaptomethylthio)propane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, trimethylolpropane tris(3-mercaptopropionate), trimethylolethane tris(2-mercaptoacetate), pentaerythritol tetrakis(2-mercaptoacetate) and/or pentaerythritol tetrakis(3-mercaptopropionate).

9. Composition according to any of the preceding claims, **characterized in that** as component C) catalysts, surface-active agents, UV stabilizers, antioxidants, fragrances, mold release agents, fillers and/or pigments are employed.

10. Composition according to Claim 9, **characterized in that** as mold release agents phosphate esters are employed, in particular mono- and/or dialkyl phosphates having 2 to 18 carbon atoms in the alkyl radical, preferably 8 to 12 carbon atoms.

11. Composition according to Claim 9 or 10, **characterized in that** as mold release agents mono- and/or dialkoxyalkyl phosphates having 2 to 12 carbon atoms in the alkoxyalkyl radical and up to three ether groups per alkoxyalkyl radical, in particular 4 to 10 carbon atoms.

12. Process for producing transparent polythiourethane articles by reaction of a composition containing or consisting of
A) a polyisocyanate component containing at least one polyisocyanate having a functionality of isocyanate groups of at least 2 per molecule,
B) a thiol component containing at least one polythiol having a functionality of thiol groups of at least 2 per molecule
and optionally
C) auxiliary and additive agents,
wherein the ratio of isocyanate groups to isocyanate-reactive groups is 0.5 : 1 to 2.0 : 1, **characterized in that** the composition further contains
D) at least one aromatic nitrile.

13. Compact and transparent polythiourethane article obtainable by reaction of the components of a composition according to any of Claims 1 to 11, wherein the polythiourethane article is in particular a glass-replacement part, an optical, optoelectronic or electronic component part, an optical lens or a spectacle glass.

14. Use of aromatic nitriles for producing transparent polythiourethane articles.

## Revendications

1. Composition destinée à la production de corps polythio-uréthane transparents contenant ou consistant en
A) un composant polyisocyanate contenant au moins un polyisocyanate ayant une fonctionnalité de groupes isocyanate d'au moins 2 par molécule,
B) un composant thiol contenant au moins un polythiol ayant une fonctionnalité de groupes thiol d'au moins 2 par molécule,
ainsi qu'éventuellement
C) des adjuvants et additifs,
le rapport des groupes isocyanate aux groupes réactifs vis-à-vis d'isocyanates valant de 0,5 : 1 à 2,0 : 1.
**caractérisée en ce que** la composition contient en outre
D) au moins un nitrile aromatique.

2. Composition selon la revendication 1, **caractérisée en ce que** la composition contient 0,0025 à 10 % en poids, en particulier 0,005 à 5 % en poids, de nitrile aromatique, par rapport à la composition totale.

3. Composition selon la revendication 1 ou 2, **caractérisée en ce que** le nitrile contient au moins un autre groupe fonctionnel, en particulier un groupe isocyanate.

4. Composition selon l'une quelconque des revendications précédentes, **caractérisée en ce que** le nitrile est choisi parmi le benzonitrile, le 3-(isocyanatométhyl)benzonitrile, le 4-(isocyanato-méthyl)benzonitrile, le 3-(chlorométhyl)benzonitrile, le 4-(chlorométhyl)benzonitrile ou des mélanges de ceux-ci, en particulier le benzonitrile, le 3-(isocyanatométhyl)benzonitrile et/ou le 4-(isocyanatométhyl)benzonitrile.

5. Composition selon l'une quelconque des revendications précédentes, **caractérisée en ce que** le polyisocyanate est choisi parmi le 1,3-bis(isocyanato-méthyl)benzène (1,3 XDI), le 1,4-bis(isocyanatométhyl)-benzène (1,4 XDI), le 2,5-bis(isocyanatométhyl)-bicyclo[2.2.1]heptane, le 2,6-bis(isocyanatométhyl)-bicyclo[2.2.1]heptane, le 1,4-bis(isocyanatométhyl)-cyclohexane, le 1,3-bis(isocyanatométhyl)cyclohexane, le 2,2'-diisocyanatodicyclohexylméthane, le 2,4'-diisocyanatodicyclohexylméthane, le 4,4'-diisocyanatodicyclohexylméthane (H12 MDI), le 1-isocyanato-3,3,5-triméthyl-5-isocyanatométhylcyclohexane (isophorone-diisocyanate ; IPDI) ou des mélanges de ceux-ci, de façon particulièrement préférée le 1,3-bis(isocyanatométhyl)benzène (1,3 XDI) et/ou le 1,4-bis(isocyanatométhyl)benzène (1,4 XDI).

6. Composition selon l'une quelconque des revendications précédentes, **caractérisée en ce que** le polyisocyanate est un polyisocyanate aromatique et le nitrile est dérivé de la même polyamine que le polyisocyanate.

7. Composition selon la revendication 5 ou 6, **caractérisée en ce que** le polyisocyanate choisi est le 1,3-bis(isocyanatométhyl)benzène (1,3 XDI) et le nitrile choisi est le 3-(isocyanatométhyl)benzonitrile et/ou le polyisocyanate choisi est le 1,4-bis(isocyanatométhyl)benzène (1,4 XDI) et le nitrile choisi est le 4-(isocyanatométhyl)benzonitrile.

8. Composition selon l'une quelconque des revendications précédentes, **caractérisée en ce que** le polythiol est choisi parmi le 4-mercaptométhyl-1,8-dimercapto-3,6-dithiaoctane, le 2,5-bismercaptométhyl-1,4-dithiane, le 1,1,3,3-tétrakis(mercaptométhyl-thio)propane, le 5,7-dimercaptométhyl-1,11-dimercapto-3,6,9-trithia-undécane, le 4,7-dimercaptométhyl-1,11-dimercapto-3,6,9-trithia-undécane, le 4,8-dimercapto-méthyl-1,11-dimercapto-3,6,9-trithia-undécane, le tris(3-mercaptopropionate) de triméthylolpropane, le tris(2-mercaptoacétate) de triméthyloléthane, le tétrakis(2-mercaptoacétate) de pentaérythritol et/ou le tétrakis(3-mercaptopropionate) de pentaérythritol.

9. Composition selon l'une quelconque des revendications précédentes, **caractérisée en ce qu'**en tant que composant C) on utilise des catalyseurs, des agents tensioactifs, des stabilisants UV, des antioxydants, des parfums, des agents de démoulage, des charges et/ou des pigments.

10. Composition selon la revendication 9, **caractérisée en ce qu'**en tant qu'agents de démoulage on utilise des esters d'acide phosphorique, en particulier des phosphates de mono- et/ou dialkyle ayant de 2 à 18 atomes de carbone, de préférence de 8 à 12 atomes de carbone, dans le radical alkyle.

11. Composition selon la revendication 9 ou 10, **caractérisée en ce qu'**en tant qu'agents de démoulage on utilise des phosphates de mono- et/ou dialcoxyalkyle ayant de 2 à 12 atomes de carbone, en particulier 4 à 10 atomes de carbone, dans le radical alcoxyalkyle et comportant jusqu'à trois groupes éther par radical alcoxyalkyle.

12. Procédé pour la production de corps polythio-uréthane transparents, par mise en réaction d'une composition contenant ou consistant en
A) un composant polyisocyanate contenant au moins un polyisocyanate ayant une fonctionnalité de groupes isocyanate d'au moins 2 par molécule,
B) un composant thiol contenant au moins un polythiol ayant une fonctionnalité de groupes thiol d'au moins 2 par molécule,
ainsi qu'éventuellement
C) des adjuvants et additifs,
le rapport des groupes isocyanate aux groupes réactifs vis-à-vis d'isocyanates valant de 0,5 : 1 à 2,0 : 1.
**caractérisé en ce que** la composition contient en outre
D) au moins un nitrile aromatique.

13. Corps polythio-uréthane transparent compact pouvant être obtenu par mise en réaction des composants d'une composition selon l'une quelconque des revendications 1 à 11, le corps polythio-uréthane consistant en particulier en une pièce remplaçant le verre, un composant optique, optoélectronique ou électronique, en lentilles optiques ou verres de lunettes.

14. Utilisation de nitriles aromatiques pour la production de corps polythio-uréthane transparents.
